# EUROPEAN PATENT APPLICATION

(11) **EP 1 384 714 A1**
(43) Date of publication of application: **28.01.2004**
(21) Application number: 02713284.4
(22) Date of filing: 02.04.2002
(51) Int. Cl.: C07D 231/14, A01N 43/56

(54) **PYRAZOLECARBOXAMIDE DERIVATIVE, INTERMEDIATE THEREFOR, AND PEST CONTROL AGENT CONTAINING THE SAME AS ACTIVE INGREDIENT**

(30) Priority: 06.04.2001 JP 2001108116
(71) Applicant: Nihon Nohyaku Co., Ltd., Tokyo 103-8236 (JP)
(72) Inventor: OKADA, Itaru, c/o Mitsubishi Chemical Corporation, Yokohama-shi, Kanagawa227-8502 (JP); TAKIZAWA, Eiji, Mitsubishi Chemical Corporation, Yokohama-shi, Kanagawa 227-8502 (JP); KIKUTAKE, Kazuhiko, Mitsubishi Chemical Corp., Yokohama-shi, Kanagawa 227-8502 (JP); FUKUCHI, Toshiki, Mitsubishi Chemical Corporation, Yokohama-shi, Kanagawa 227-8502 (JP)
(74) Representative: HOFFMANN EITLE
(86) International application number: PCT/JP2002/003308
(87) International publication number: WO 2002/083647

(57) **Abstract**

The present invention provides novel compounds with fungicidal and bactericidal activity as well as insecticidal and miticidal activity, which are useful for controlling hazardous biological organisms and belong to pyrazolecarboxamides represented by the following formula (I) : 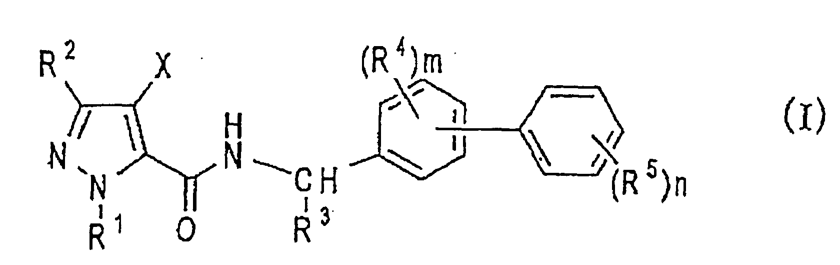 wherein R¹ represents C₁-C₅ alkyl; R² represents C₁-C₅ alkyl, C₁-C₅ haloalkyl, or the like; R³ represents hydrogen or C₁-C₃ alkyl; R⁴ represents halogen, C₁-C₅ alkyl or the like; m is an integer of 0 to 4; R⁵ represents alkyl, haloalkyl, alkoxy, haloalkoxy or the like; n represents an integer of 1 to 5; and X represents hydrogen, C₁-C₃ alkyl, C₁-C₃ alkoxy, halogen, C₁-C₃ haloalkoxy or nitro.

## Description

### Technical Field

The present invention relates to novel pyrazolecarboxamides and intermediates therefor, and pest control agents comprising the same as an active ingredient. The novel pyrazolecarboxamides in accordance with the present invention are useful as bactericides and fungicides, insecticides and miticides for agriculture, and horticulture and floriculture.

### Background of the Invention

In the field of agriculture as well as horticulture and floriculture, various fungicides and bactericides, insecticides and miticides have been developed and used in a practical sense for the purpose of controlling various hazardous insects and plant diseases. However, it cannot be said that agricultural chemicals for common use in the related art have been satisfactory in terms of, for example, effect, spectrum and residual effect or have satisfied the demand to reduce the number and chemical dose to be applied.

Additionally, the emergence of hazardous insects and plant diseases with acquired resistance against agricultural chemicals for common use in the related art is now problematic. For the cultivation of vegetables, fruit trees, flowers, teas, wheat, barley, oats, etc., rice plants and the like, various hazardous insects with acquired resistance against diverse types of insecticides of, for example, carbamate-series, pyrethroid-series, benzoyl urea-series, organic chloride-series, and organic phosphorus-series and various pathological microorganisms and bacteria with acquired resistance against various types of fungicides and bactericides of, for example, triazole-series, imidazole-series, pyrimidine-series, benzimidazole-series, dicarboximide-series, and phenylamide-series have emerged in various local regions. Therefore, it has been very tough increasingly year by year to control these various hazardous insects and pathological microorganisms and bacteria causing plant diseases, because of such emergence of these resistant hazardous insects.

Thus, it has been consistently desired the development of a novel agricultural chemical of which a lower chemical dose can express a sufficient effect on the control of various pathological microorganisms and bacteria and hazardous insects with acquired resistance against insecticides and fungicides and bactericides for agriculture and horticulture and floriculture in the related art and which shows less adverse effects on environment.

The present inventors found that N-benzylpyrazole-5-carboxamide derivatives had insecticidal and miticidal activity. Specifically, the publication of JP-A-64-25763 discloses the following compound with insecticidal and miticidal activity, and the like.

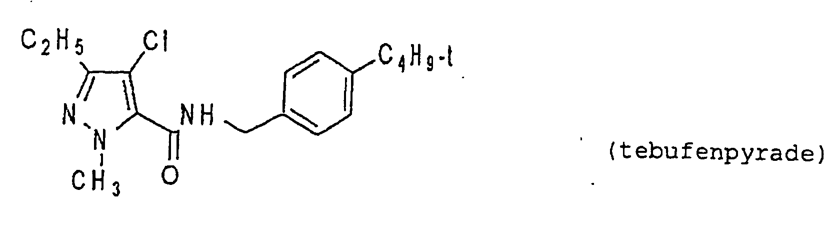

Additionally, the publication of JP-A-3-81266 discloses that the following compound with phenoxy and the like have insecticidal and miticidal activity.

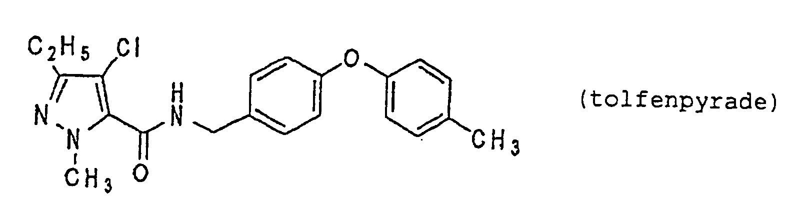

However, Compound (III) with a structure similar to the compound of the present invention as disclosed in JP-A-64-25763 is so insufficient in view of the insecticidal and miticidal activity that the practical applicability of the compound is still low.

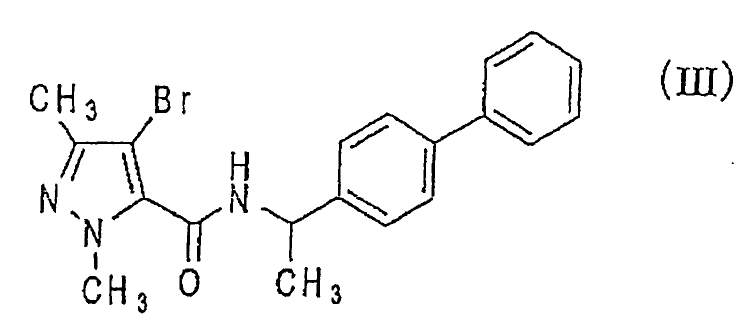

Meanwhile, a compound with both fungicidal and bactericidal activity and insecticidal and miticidal activity is very useful for use in agriculture and horticulture and floriculture. Regarding fungicidal and bactericidal activity, it is known that only a compound with specific substituents among compounds described in JP-A-64-25763 has fungicidal and bactericidal activity (JP-A-3-206079).

An object of the present invention to provide a new substance with a great effect on the control of various pathological microorganisms and bacteria and with usefulness in the control of insects and mites, particularly a highly safe substance with a great effect on the control of various hazardous insects and pathological bacteria causing plant diseases with acquired resistance against fungicides and bactericides and insecticides in the related art, because such a lower chemical dose thereof can express these effects that problems of residual toxicity and environmental pollution can be reduced.

### Disclosure of the Invention

The inventors have investigated the problems described above. Consequently, the inventors have found that novel pyrazolecarboxamides with a biphenyl backbone with substituents and with a specific combination of substituents have fungicidal and bactericidal activity and insecticidal and miticidal activity meeting the features described above. Thus, the present invention has been achieved.

The present invention relates to a pyrazolecarboxamide represented by the following formula (I) : 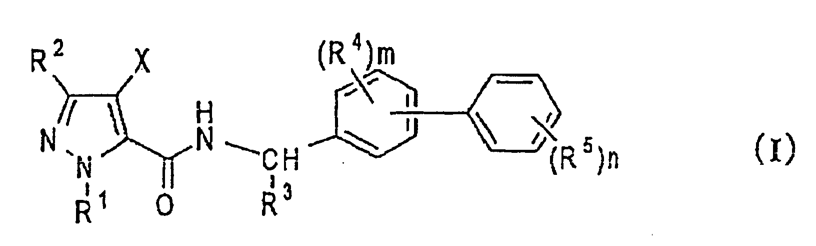 wherein R¹ represents C₁-C₅ alkyl,
R² represents C₁-C₅ alkyl, C₁-C₅ haloalkyl, C₁-C₅ alkoxy or C₁-C₅ haloalkoxy,
R³ represents hydrogen or C₁-C₃ alkyl,
R⁴ represents C₁-C₅ alkyl, C₁-C₅ haloalkyl, C₁-C₅ alkoxy, C₁-C₅ haloalkoxy or halogen,
m is an integer of 0 to 4;
R⁵ represents alkyl, haloalkyl, alkoxy, haloalkoxy, alkylthio, haloalkylthio, alkylsulfinyl, haloalkylsulfinyl, alkylsulfonyl, haloalkylsulfonyl, halogen, cyano, formyl, vinyl, alkoxycarbonyl, haloalkoxycarbonyl, acetyl, hydroxycarbonyl, alkylcarbamoyl or a group represented by 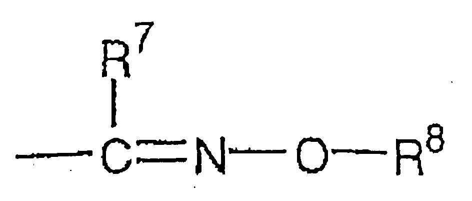 wherein R⁷ and R⁸ each represents hydrogen or C₁-C₅ alkyl,
n represents an integer of 1 to 5,
X represents hydrogen, C₁-C₃ alkyl, C₁-C₃ alkoxy, halogen, C₁-C₃ haloalkoxy or nitro, or
R² and X may be taken together to form 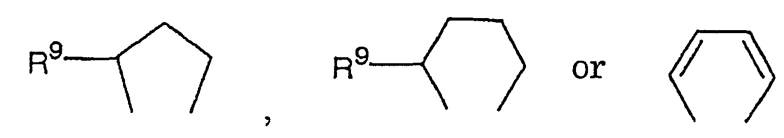 wherein R⁹ represents hydrogen or C₁-C₃ alkyl,
a pest control agents comprising the pyrazolecarboxamide as an active ingredient,
a phenylbenzylamine as an intermediate for the pyrazolecarboxamide, which is represented by the following formula (II): 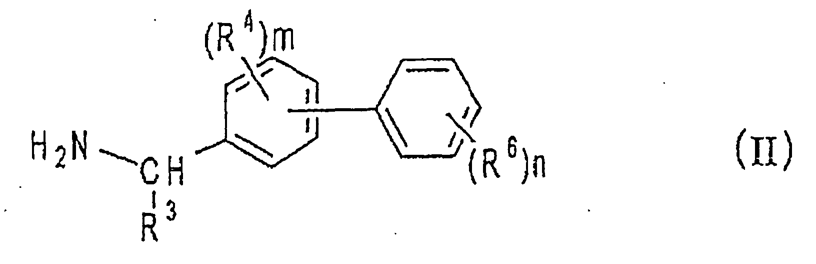 wherein R³, R⁴, m and n have the same meanings as defined in formula (I); R⁶ represents haloalkyl or haloalkoxy, and
an acid addition salt thereof.

### Best Mode for Carrying Out the Invention

The present invention is now described in detail below.

### Pyrazolecarboxamides:

In the pyrazolecarboxamides represented by formula (I) in accordance with the present invention, the substituent R¹ represents a linear or branched C₁-C₅ alkyl such as methyl, ethyl, n-propyl, isopropyl, n-butyl, *sec*-butyl, isobutyl, *tert*-butyl, n-pentyl, 2-methylbutyl, 3-methylbutyl, isopentyl, 2-ethylpropyl, neopentyl, 2,2-dimethylpropyl, or 2-methylisobutyl. Among these, methyl is preferable as the substituent R¹.

The substituent R² represents linear, branched or cyclic C₁-C₅ alkyl such as methyl, ethyl, n-propyl, isopropyl, n-butyl, *sec*-butyl, isobutyl, *tert*-butyl, n-pentyl, 2-methylbutyl, 3-methylbutyl, isopentyl, 2-ethylpropyl, neopentyl, 2,2-dimethylpropyl, 2-methylisobutyl, or cyclopropyl; linear or branched C₁-C₅ haloalkyl such as fluoromethyl, chloromethyl, bromomethyl, difluoromethyl, difluoroethyl, trifluoromethyl, trichloromethyl, tribromomethyl, 2-fluoroethyl, 2,2,2-trifluoroethyl, 1-(trifluoromethyl)ethyl, 1,1,2,3,3,3-hexafluoropropyl, 2,2,3,3,3-pentafluoropropyl, 2,2,3,3-tetrafluoropropyl, 2,2,3,3,4,4,4-heptafluorobutyl, or 2,2,3,3,4,4,5,5,5-nonafluoropentyl; linear or branched C₁-C₅ alkoxy such as methoxy, ethoxy, n-propoxy, isopropoxy, n-butoxy, *sec*-butoxy, isobutoxy, *tert*-butoxy, n-pentoxy, 2-methylbutoxy, 3-methylbutoxy, isopentoxy, 2-ethylpropoxy, neopentoxy, 2,2-dimethylpropoxy, or 2-methylisobutoxy; or linear or branched C₁-C₅ haloalkoxy such as difluoromethoxy, trifluoromethoxy, fluoromethoxy, trichloromethoxy, tribromomethoxy, 2-fluoroethoxy, 2,2,2-trifluoroethoxy, 2,2,2-trichloroethoxy, 1-(trifluoromethyl)ethoxy, 1,1,2,3,3,3-hexafluoropropoxy, 2,2,3,3,3-pentafluoropropoxy, or 2, 2, 3,3-tetrafluoropropoxy. Among these, linear C₁-C₃ alkyl is preferable as the substituent R². Ethyl is particularly preferable.

The substituent R³ represents hydrogen; or linear or branched C₁-C₃ alkyl such as methyl, ethyl, n-propyl, or isopropyl. Among these, hydrogen is preferable as the substituent R³.

The substituent R⁴ represents linear or branched C₁-C₅ alkyl such as methyl, ethyl, n-propyl, isopropyl, n-butyl, *sec*-butyl, isobutyl, *tert-*butyl, n-pentyl, 2-methylbutyl, 3-methylbutyl, isopentyl, 2-ethylpropyl, neopentyl, 2,2-dimethylpropyl, or 2-methylisobutyl; linear or branched C₁-C₅ haloalkyl such as fluoromethyl, chloromethyl, bromomethyl, difluoromethyl, difluoroethyl, trifluoromethyl, trichloromethyl, tribromomethyl, 2-fluoroethyl, 2,2,2-trifluoroethyl, 1-(trifluoromethyl)ethyl, 1,1,2,3,3,3-hexafluoropropyl, 2,2,3,3,3-pentafluoropropyl, 2,2,3,3-tetrafluoropropyl, 2,2,3,3,4,4,4-heptafluorobutyl, or 2,2,3,3,4,4,5,5,5-nonafluoropentyl; linear or branched C₁-C₅ alkoxy such as methoxy, ethoxy, n-propoxy, isopropoxy, n-butoxy, *sec*-butoxy, isobutoxy, *tert*-butoxy, n-pentoxy, 2-methylbutoxy, 3-methylbutoxy, isopentoxy, 2-ethylpropoxy, neopentoxy, 2,2-dimethylpropoxy, or 2-methylisobutoxy; linear or branched C₁-C₅ haloalkoxy such as difluoromethoxy, trifluoromethoxy, fluoromethoxy, trichloromethoxy, tribromomethoxy, 2-fluoroethoxy, 2,2,2-trifluoroethoxy, 2,2,2-trichloroethoxy, 1-(trifluoromethyl)ethoxy, 1,1,2,3,3,3-hexafluoropropoxy, 2, 2, 3, 3, 3-pentafluoropropoxy, or 2,2,3,3-tetrafluoropropoxy; or halogen such as fluorine, chlorine, bromine or iodine. Among these, halogen is preferable as the substituent R⁴.

The substituent R⁵ represents linear or branched alkyl such as methyl, ethyl, n-propyl, isopropyl, cyclopropyl, n-butyl, *sec*-butyl, isobutyl, *tert*-butyl, n-pentyl, 2-methylbutyl, 3-methylbutyl, isopentyl, 2-ethylpropyl, neopentyl, 2,2-dimethylpropyl, 2-methylisobutyl, n-hexyl, n-heptyl or n-octyl; linear or branched haloalkyls such as fluoromethyl, chloromethyl, bromomethyl, difluoromethyl, difluoroethyl, trifluoromethyl, trichloromethyl, tribromomethyl, 2-fluoroethyl, 2,2,2-trifluoroethyl, 1-(trifluoromethyl)ethyl, 1,1,2,3,3,3-hexafluoropropyl, 2,2,3,3,3-pentafluoropropyl, 2,2,3,3-tetrafluoropropyl, 2,2,3,3,4,4,4-heptafluoropropyl, or 2,2,3,3,4,4,5,5,5-nonafluoropentyl; linear or branched alkoxys such as methoxy, ethoxy, n-propoxy, isopropoxy, n-butoxy, *sec*-butoxy, isobutoxy, *tert*-butoxy, n-pentoxy, 2-methylbutoxy, 3-methylbutoxy, isopentoxy, 2-ethylpropoxy, neopentoxy, 2,2-dimethylpropoxy, 2-methylisobutoxy, n-hexyloxy, n-heptyloxy, or n-octyloxy; linear or branched haloalkoxy such as difluoromethoxy, trifluoromethoxy, fluoromethoxy, trichloromethoxy, tribromomethoxy, 2-fluoroethoxy, 2,2,2-trifluoroethoxy, 2,2,2-trichloroethoxy, 1-(trifluoromethyl)ethoxy, 1,1,2,3,3,3-hexafluoropropoxy, 2, 2, 3, 3, 3-pentafluoropropoxy, 2,2,3,3-tetrafluoropropoxy, or 2,2,3,3,4,4,4-heptafluorobutoxy; linear or branched alkylthio such as methylthio, ethylthio, propylthio, isopropylthio, butylthio, sec-butylthio, isobutylthio, tert-butylthio, pentylthio, 2-methylbutylthio, 3-methylbutylthio, isopentylthio, 2-ethylpropylthio, neopentylthio, 2,2-dimethylpropylthio, or 2-methylisobutylthio; haloalkylthio such as trifluoromethylthio, perfluoroethylthio, perfluoropropylthio, or 2,2,2-trifluoroethylthio; linear or branched alkylsulfinyl such as methylsulfinyl, ethylsulfinyl, propylsulfinyl, isopropylsulfinyl, butylsulfinyl, *sec*-butylsulfinyl, isobutylsulfinyl, *tert*-butylsulfinyl, pentylsulfinyl, 2-methylbutylsulfinyl, 3-methylbutylsulfinyl, isopentylsulfinyl, 2-ethylpropylsulfinyl, neopentylsulfinyl, 2,2-dimethylpropylsulfinyl or 2-methylisobutylsulfinyl; haloalkylsulfinyl such as trifluoromethylsulfinyl or 2,2,2-trifluoroethylsulfinyl; linear or branched alkylsulfonyl such as methylsulfonyl, ethylsulfonyl, propylsulfonyl, isopropylsulfonyl, butylsulfonyl, sec-butylsulfonyl, isobutylsulfonyl, tert-butylsulfonyl, pentylsulfonyl, 2-methylbutylsulfonyl, 3-methylbutylsulfonyl, isopentylsulfonyl, 2-ethylpropylsulfonyl, neopentylsulfonyl, 2,2-dimethylpropylsulfonyl, or 2-methylisobutylsulfonyl; haloalkylsulfonyl such as trifluorosulfonyl or 2,2,2-trifluoroethylsulfonyl; halogen such as fluorine, chlorine, bromine or iodine; cyano; formyl; vinyl; linear or branched alkoxycarbonyl such as methoxycarbonyl, ethoxycarbonyl, n-propoxycarbonyl, isopropoxycarbonyl or butoxycarbonyl; haloalkoxycarbonyl such as 2,2,2-trifluoromethoxycarbonyl or 2,2,3,3,3-pentafluorpropoxycarbonyl; acetyl; hydroxycarbonyl; alkylcarbamoyl such as methylcarbamoyl, ethylcarbamoyl or propylcarbamoyl; or a group represented by

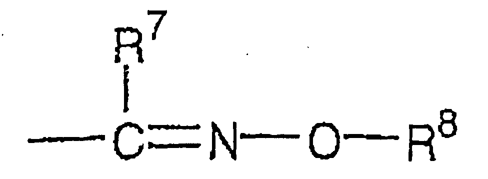

Herein, the above R⁷ and R⁸ each represents hydrogen; or linear or branched C₁-C₅ alkyl such as methyl, ethyl, n-propyl, isopropyl, n-butyl, *sec*-butyl, isobutyl, or pentyl.

In the above R⁵, the alkyl and alkoxy have preferably C₁-C₈; the haloalkyl, haloalkoxy, alkylthio, haloalkylthio, alkylsulfinyl, haloalkylsulfinyl, alkylsulfonyl and haloalkylsulfonyl have preferably C₁-C₅. The alkoxycarbonyl, haloalkoxycarbonyls, alkylcarbamoyls and group represented by 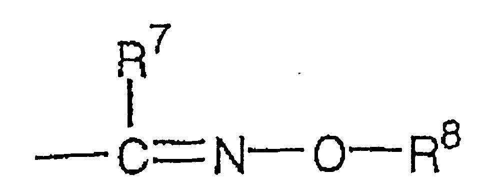 have preferably C₂-C₈, more preferably C₂-C₅.

Among these, the substituent R⁵ is preferably haloalkyl or haloalkoxy, more preferably C₁-C₅ haloalkyl or C₁-C₅ haloalkoxy, particularly preferably trifluoromethoxy or difluoromethoxy. Additionally, the substituent is preferably positioned at the 4-position.

The substituent X represents hydrogen; linear or branched C₁-C₃ alkyl such as methyl, ethyl, n-propyl, and isopropyl; linear or branched C₁-C₃ alkoxy such as methoxy, ethoxy, n-propoxy, or isopropoxy; halogen such as fluorine, chlorine, bromine, or iodine; C₁-C₃ haloalkoxy such as difluoromethoxy, 2,2,2-trifluoroethoxy, or perfluoropropoxy; or nitro. Among these, the substituent X is preferably hydrogen.

Furthermore, R⁹ in the group 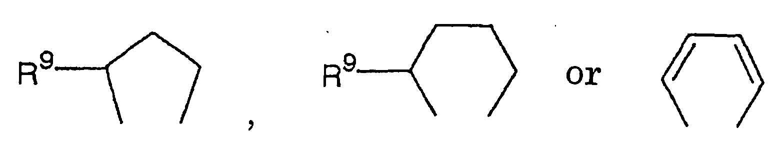 formed by R² and X together represents hydrogen; or C₁-C₃ alkyl such as methyl, ethyl, n-propyl or isopropyl.

m is an integer of 0 to 4, preferably 0 to 2; and n is an integer of 1 to 5, preferably 1 or 2.

### Phenylbenzylamines:

Furthermore, the phenylbenzylamines represented by formula (II) and acid addition salts thereof in accordance with the present invention are novel intermediates for producing the pyrazolecarboxamides.

Herein, the substituents R³ and R⁴, m and n have the same meanings as defined in formula (I).

The substituent R⁶ represents haloalkyl such as fluoromethyl, chloromethyl, bromomethyl, difluoromethyl, difluoroethyl, trifluoromethyl, trichloromethyl, tribromomethyl, 2-fluoroethyl, 2,2,2-trifluoroethyl, 1-(trifluoromethyl)ethyl, 1,1,2,3,3,3-hexafluoropropyl, 2,2,3,3,3-pentafluoropropyl, 2,2,3,3-tetrafluoropropyl, 2, 2, 3,3,4,4, 4-heptafluorobutyl, or 2,2,3,3,4,4,5,5,5-nonafluoropentyl; or haloalkoxys such as difluoromethoxy, trifluoromethoxy, fluoromethoxy, trichloromethoxy, tribromomethoxy, 2-fluoroethoxy, 2,2,2-trifluoroethoxy, 2,2,2-trichloroethoxy, 1-(trifluoromethyl)ethoxy, 1,1,2,3,3,3-hexafluoropropoxy, 2,2,3,3,3-pentafluoropropxy, 2,2,3,3-tetrafluoropropoxy, or 2,2,3,3,4,4,4-heptafluorobutoxy. The haloalkyl and haloalkoxy have preferably C₁-C₅.

The acid to be added to the phenylbenzylamines represented by formula (II) in accordance with the present invention includes carboxylic acid such as acetic acid, propionic acid, butyric acid, oxalic acid, adipic acid, dodecanedioic acid, lauric acid, stearic acid, trifluoroacetic acid, fumaric acid, maleic acid, benzoic acid, or phthalic acid; sulfonate such as methanesulfonic acid, 1,3-propanedisulfonic acid, p-toluenesulfonic acid, or dodecylbenzenesulfonic acid; and inorganic acid such as hydrochloric acid, sulfuric acid, nitric acid, or carbonic acid.

### Production process:

The compound represented by formula (I) in accordance with the present invention is a novel compound and can be produced, for example, by the following reaction scheme. 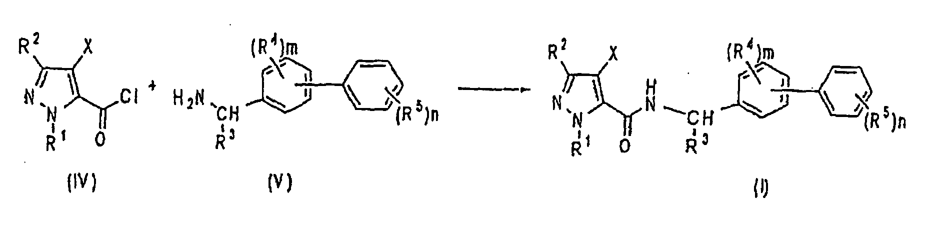 (In the formula, R¹, R², R³, R⁴, R⁵, X, m and n have the same meanings as described in formula (I).)

In other words, pyrazolecarboxylic acid chloride (IV) and phenylbenzylamine (V) are allowed to react in the presence or absence of a base, preferably using a solvent at -10 to 50°C, preferably 0 to 25°C.

As the solvent, any solvent can be used, so long as it does not have direct influence on the reaction. The solvent includes aromatic hydrocarbons such as benzene, toluene and xylene; ketones such as acetone, methyl ethyl ketone, and methyl isobutyl ketone; halogenated hydrocarbons such as chloroform and dichloromethane; water; esters such as methyl acetate and ethyl acetate; and polar solvents such as tetrahydrofuran, acetonitrile, dioxane, N,N-dimethylformamide, N-methylpyrrolidone, dimethyl sulfoxide, and pyridine.

The base includes alkali metal hydrides such as sodium hydride; alkali metal hydroxides such as sodium hydroxide and potassium hydroxide; alkali metal carbonates such as sodium carbonate and potassium carbonate; inorganic salts; and amines such as pyridine and triethylamine.

In order to isolate the objective compound of formula (I) after the reaction, the following procedures are carried out in case of using a solvent soluble in water: distilling off the solvent under reduced pressure, adding water to the resulting residue, extracting the residue in aromatic hydrocarbons insoluble in water such as benzene, toluene and xylene, halogenated hydrocarbons such as chloroform and dichloromethane, and esters such as ethyl acetate, rinsing the extract with a saturated aqueous sodium chloride solution, drying the extract over desiccants such as anhydrous sodium sulfate or anhydrous magnesium sulfate and then distilling off the solvents under reduced pressure. When a solvent insoluble in water is used, water is added to the reaction mixture, followed by separation; the resulting organic layer is rinsed with a saturated aqueous sodium chloride solution and dried over desiccants such as anhydrous sodium sulfate or magnesium sulfate, from which the solvent is distilled off under reduced pressure. The residue recovered after distillation of the solvent is purified by recrystallization, suspending and rinsing, and column chromatography, so that the objective compound represented by formula (I) can be recovered.

The compound represented by formula (IV) as one raw material can be synthetically prepared, for example, by the method described in JP-A-64-25763.

The compound represented by formula (V) as one raw material can be synthetically prepared, for example, by the following method.

### (1) Reduction of phenylbenzonitriles

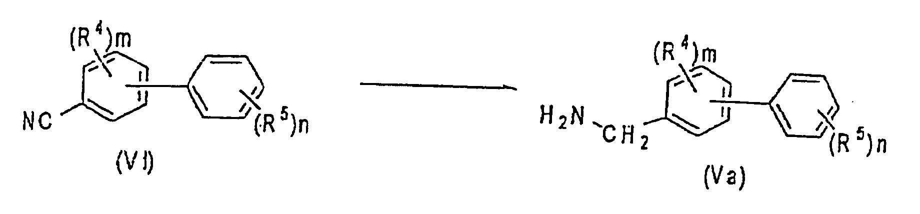

( In the formula, R⁴, R⁵, m and n have the same meanings as described in formula (I).)

Phenylbenzonitrile (VI) and hydrogen are allowed to react in the presence of a metal catalyst and aqueous ammonia, preferably using an organic solvent, at 0 to 100°C, preferably 25 to 50°C.

As the organic solvent, any organic solvent can be used, so long as it does not have directly influence on the reaction. The organic solvent includes alcohols such as methanol, ethanol and isopropanol; aromatic hydrocarbons such as benzene, toluene and xylene; ketones such as acetone, methyl ethyl ketone, and methyl isobutyl ketone; halogenated hydrocarbons such as chloroform and dichloromethane; water; esters such as methyl acetate and ethyl acetate; or polar solvents such as tetrahydrofuran, acetonitrile, dioxane, N,N-dimethylformamide, N-methylpyrrolidone, dimethyl sulfoxide, and pyridine.

The metal catalyst includes Raney-nickel, palladium carbon, and platinum oxide.

In order to isolate the objective compound of formula (Va) after the reaction, the metal catalyst is filtered off from the reaction mixture, and the solvent is distilled off from the resulting filtrate under reduced pressure. The resulting product may be sufficiently pure as it is, but the product can be purified by means of evaporation and column chromatography, to recover a pure product.

Phenylbenzonitrile (VI) can be synthetically prepared by reaction of the corresponding haloallene with phenylboronic acid in the presence of a zero-valent palladium catalyst and a base according to known methods, for example, the method described in *Synthetic Communications*, Vol. 11, page 513 (1981).

### (2) Reduction of oximes

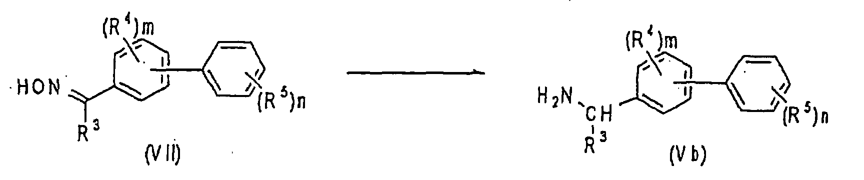

Oxime (VII) and hydrogen are allowed to react in the presence of a metal catalyst and aqueous ammonia, preferably using an organic solvent, at 0 to 100 °C, preferably 25 to 50 °C.

As the organic solvent, any organic solvent can be used, so long as it has not direct influence on the reaction. The organic solvent includes alcohols such as methanol, ethanol and isopropanol; aromatic hydrocarbons such as benzene, toluene and xylene; ketones such as acetone, methyl ethyl ketone, and methyl isobutyl ketone; halogenated hydrocarbons such as chloroform and dichloromethane; water; esters such as methyl acetate and ethyl acetate; and polar solvents such as tetrahydrofuran, acetonitrile, dioxane, N,N-dimethylformamide, N-methylpyrrolidone, dimethyl sulfoxide, and pyridine.

The metal catalyst includes Raney-nickel, palladium carbon, and platinum oxide.

In order to isolate the objective compound of formula (Vb) after the reaction, the metal catalyst is filtered off from the reaction mixture, and the solvent is distilled off from the resulting filtrate under reduced pressure .

Oxime (VII) can be synthetically prepared by reaction of the corresponding acetophenone with hydroxyamine in the presence of potassium carbonate according to known methods.

Phenylbenzylamine-acid addition salts (Vc) can be synthesized by the following method.

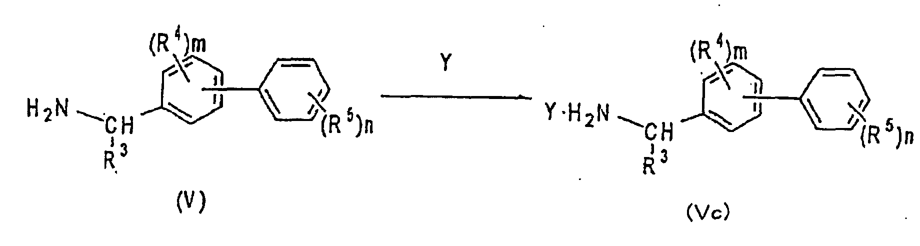

Phenylbenzylamine (V) and acid (Y) are allowed to react, preferably using a solvent, at -5 to 50°C, preferably 0 to 25°C.

As the acid, organic acid and inorganic acid can be used. The organic acid includes carboxylic acids such as acetic acid, propionic acid, butyric acid, oxalic acid, adipic acid, dodecanedioic acid, lauric acid, stearic acid, trifluoroacetic acid, fumaric acid, maleic acid, benzoic acid, and phthalic acid; and sulfonates such as methanesulfonic acid, 1,3-propanedisulfonic acid, p-toluenesulfonic acid, and dodecylbenzenesulfonic acid. The inorganic acid includes hydrochloric acid, sulfuric acid, nitric acid and carbonic acid.

As the solvent, any solvent can be used, so long as it does not have direct influence on the reaction. The organic solvent includes aromatic hydrocarbons such as benzene, toluene and xylene; ketones such as acetone, methyl ethyl ketone, and methyl isobutyl ketone; halogenated hydrocarbons such as chloroform and dichloromethane; water; esters such as methyl acetate and ethyl acetate; and polar solvents such as tetrahydrofuran, acetonitrile, dioxane, N,N-dimethylformamide, N-methylpyrrolidone, dimethyl sulfoxide, and pyridine.

After the reaction, the solvent is distilled off under reduced pressure. The resulting crystals are filtered and recovered, to synthetically prepare inorganic acid salts of benzylamine and organic acid salts thereof. The crystals are satisfactory as they are. For further purification, however, the crystals are rinsed with the organic solvents described above, to synthetically prepare benzylamine salts at high purity.

### Pest control agents:

The compound represented by formula (I) in accordance with the present invention has high fungicidal and bactericidal effects on pathological microorganisms and bacteria against plants such as *Pyricularia oryzae*, pathological microorganisms and bacteria causing powdery mildew, pathological microorganisms and bacteria causing rust, and pathological microorganisms and bacteria causing downy mildew so that the compound is useful as an active ingredient for fungicides and bactericides for agriculture and horticulture and floriculture. Since the compound of the present invention has high activity on the control of the eggs and larvae of insects of the order *Hemiptera* including leafhoppers such as *Sogatella furcifera Horvath, Nilaparvata lugens (Stal)*, and *Laodelphax stratella (Fallen); Cicadomorpha-2* such as *Bothrogonia ferruginea (Fabricius)* and *Cicadella viridis (Linnaeus);* and *Aphididae* such as *Myzus persicae*; the order *Lepidoptera* including *Spodoptera litura Fabricius, Chilo suppresalis, Cnaphalocrosis medinalis Guenee* and *Plutella xylostella (Linnaeus);* the order *Coleoptera* including *Callosobruchus chinensis*; the order *Diptera* including *Musca domestica Linnaeus, Aedes aegypti* and *Culex pipiens pallens*; the order *Orthoptera*; and the order *Acarina* including *Tetranychus urticae Koch, Tetranychus cinnabarinus (Boisduval)* and *Panonychus citri (Mcgregor)*, the compound of the present invention is useful as an active ingredient for insecticides and miticides for agriculture and horticulture and floriculture. It is needless to say that the pathological microorganisms and bacteria against plants, insects, and mites as subjects to be controlled with the compound of the present invention are not limited to those described above.

When the compound represented by formula (I) in accordance with the present invention is used for fungicides and bactericides, insecticides and/or miticides for agriculture and horticulture and floriculture, the compound may satisfactorily be used singly. However, preferably, the compound may be used in composition forms produced using agricultural auxiliary agents for common use in the art, such as fungicides and bactericides, insecticides and/or miticides for agriculture and horticulture and floriculture. The forms are preferably, for example, emulsions, hydrates, powders, flowable agents, fine granules, granules, tablets, oils, sprays, and fumes. However, the forms are not limited thereto. One or two or more forms of the compound may also be blended as active ingredients.

The agricultural auxiliary agents for use in producing the fungicides and bactericides, insecticides and/or miticides for agriculture and horticulture and floriculture are used, for example, for the improvement of the effects of fungicides and bactericides, insecticides and/or miticides for agriculture and horticulture and floriculture and for the improvement of stability and dispersibility. For example, carriers (diluents), spreading agents, emulsifiers, wet spreading agents, dispersants and disintegrators can be used.

The liquid carriers include water, aromatic hydrocarbons such as toluene and xylene, alcohols such as methanol, butanol, and glycol, ketones such as acetone, amides such as dimethylformamide, sulfoxides such as dimethyl sulfoxide, methylnaphthalene, cyclohexane, animal and vegetable oils, and fatty acid. Additionally, the solid carriers include clay, kaolin, talc, diatomaceous earth, silica, calcium carbonate, montmollionite, bentonite, feldspar, quartz, alumina, sawdust, nitrocellulose, starch and gum arabic.

As the emulsifiers and the dispersants, general surfactants can be used and include anionic surfactants, cationic surfactants, nonionic surfactants and ampholytic surfactants, such as higher alcohol sodium sulfate, stearyltrimethylammonium chloride, polyoxyethylene alkylphenyl ether, and lauryl betaine. Additionally, the following agents can also be used: spreading agents such as polyoxyethylene nonyl phenyl ether and polyoxyethylene lauryl phenyl ether; wet spreading agents such as dialkylsulfosuccinate; attaching agents such as carboxymethyl cellulose and polyvinyl alcohol; and disintegrators such as sodium lignin sulfonate and sodium lauryl sulfate.

The content of the active ingredient in the fungicides and bactericides, insecticides and/or miticides for agriculture and horticulture and floriculture in accordance with the present invention is selected within a range of 0.1 to 99.5% and is appropriately determined on the basis of various conditions for preparations and application methods. Preferably, for example, powders are produced so as to contain the active ingredient at about 0.5 to 20% by weight, preferably about 10% by weight; hydrates are produced so as to contain the active ingredient at about 1 to 90% by weight, preferably 10 to 80% by weight; emulsions are produced so as to contain the active ingredient at about 1 to 90% by weight, preferably about 10 to 40% by weight.

In the case of the emulsions, for example, solvents, surfactants and the like are mixed with the compound as the active ingredient to prepare a stock emulsion. The stock emulsion is diluted with water to a given concentration when used, and is applied. The resulting solution can be applied. In the case of the hydrates, the compound as the active ingredient, a solid carrier, a surfactant and the like are mixed together to prepare a stock solution. The stock solution is diluted with water when used, and is applied. In the case of the powders, the compound as the active ingredient, a solid carrier and the like are mixed together. The resulting mixture can be used as it is. In the case of the granules, the compound as the active ingredient, a solid carrier, a surfactant and the like are mixed together and granulated, to prepare granules, which can be used as it is. It is needless to say that the methods for preparing the preparations are not limited to those described above. One skilled in the art can select an appropriate method, depending on the type of the active ingredient, the purpose of application and the like.

In addition to the compound as the active ingredient, the fungicides and bactericides, the insecticides and/or miticides for agriculture and horticulture and floriculture in accordance with the present invention may contain any appropriate active ingredients such as other fungicides and bactericides, insecticides, miticides, herbicides, insect growth controlling agents, fertilizers, and soil modifiers. The method for applying the fungicides and bactericides, insecticides and/or miticides for agriculture and horticulture and floriculture in accordance with the present invention is not particularly limited, and spraying on stem and leaf, application on water surface, soil treatment, and seed treatment can be carried out. In the case of spraying on stem and leaf, for example, a solution within a concentration range of 5 to 1,000 ppm, preferably 10 to 500 ppm can be used at a volume of about 100 to 200 liters per 10 Ares. For application on water surface, generally, granules at 5-15% content of the active ingredient are applied at a ratio of 1 to 10 kg per 10 ares. In the case of soil treatment, a solution within a concentration range of 5 to 1,000 ppm is applied at a ratio of about 1 to 10 liters per m². In the case of seed treatment, a solution within a concentration range of 10 to 1,000 ppm is applied at a ratio of about 10 to 100 ml per kg of seeds.

The present invention is now described in the following examples in more detail. However, the scope of the present invention is not limited to the following examples.

### Examples

The present invention is now described below in detail in the following examples, Formulation Examples and test examples. However, the present invention is not limited to the examples, unless the examples are departed from the scope of the present invention.

### Example 1

### N-[4-(4-Difluoromethoxyphenyl)benzyl]-1-methyl-3-ethylpyrazole-5-carboxamide:

A mixture of 4-(4-difluoromethoxyphenyl)benzylamine (13.8 g), triethylamine (10 ml) and dichloromethane (100 ml) was cooled to 0°C to 4°C, followed by addition of a solution (20 ml) of 1-methyl-3-ethylpyrazole-5-carboxylic acid chloride (12.4 g) in dichloromethane, for agitation for 30 minutes. The reaction mixture was poured into water (200 ml), for extraction in dichloromethane. The organic layer was rinsed with a saturated aqueous sodium chloride solution and dried over anhydrous sodium sulfate. The solvent was distilled off under reduced pressure. The resulting residue was purified by silica gel chromatography, to obtain 17.0 g of Compound No. 30 described in Table-1. The melting point was 107 to 108°C.

### Example 2

According to the method in Example 1, the compounds described in Table-1 were prepared synthetically.

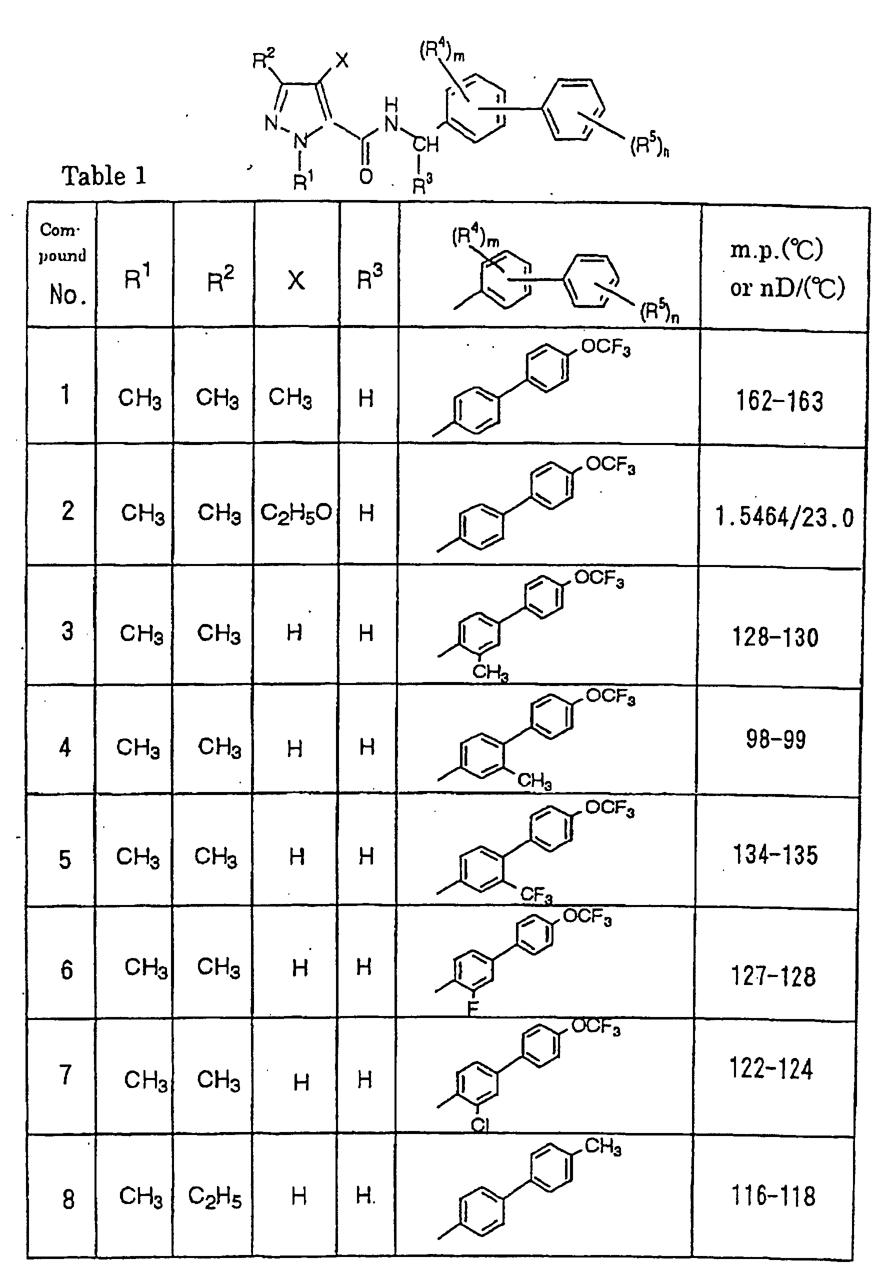

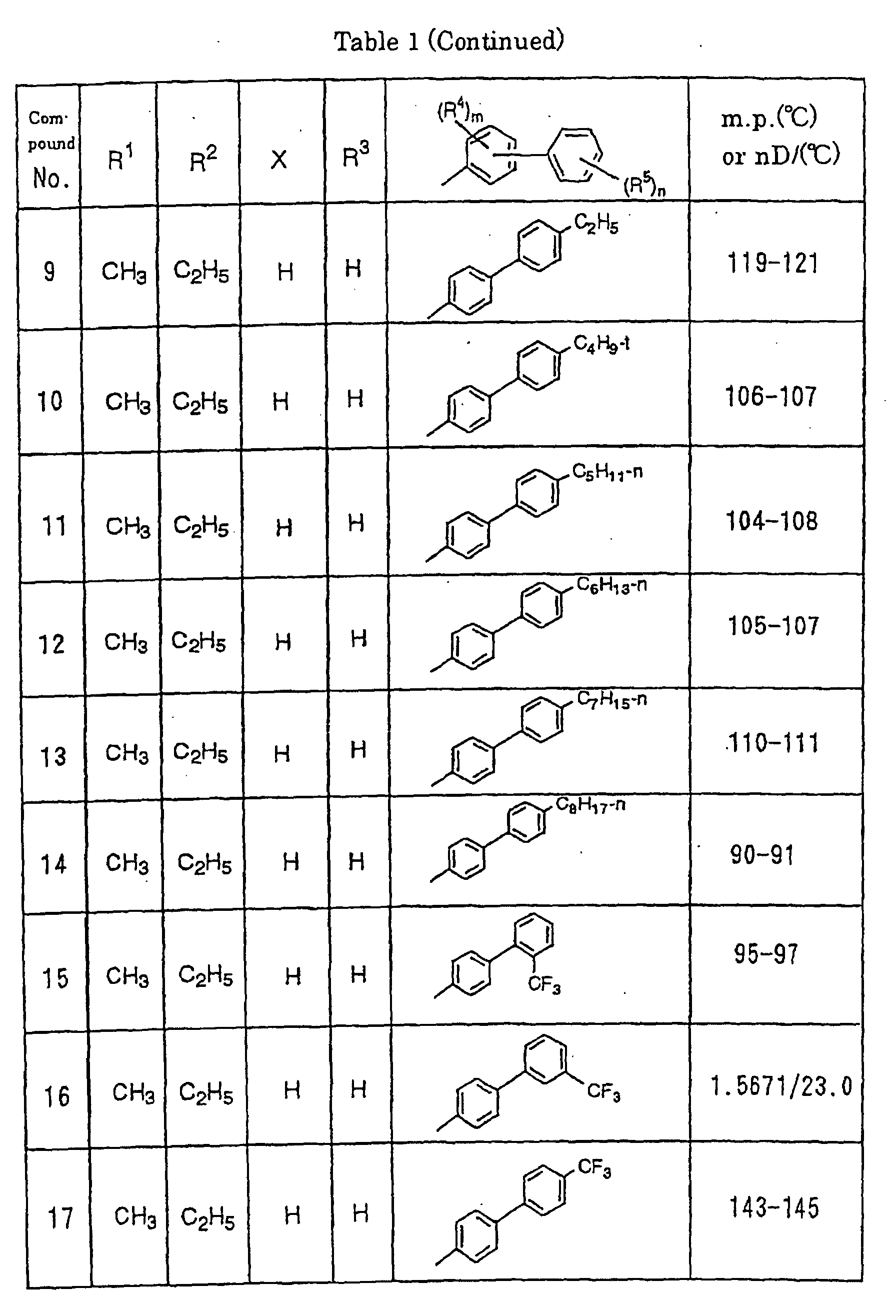

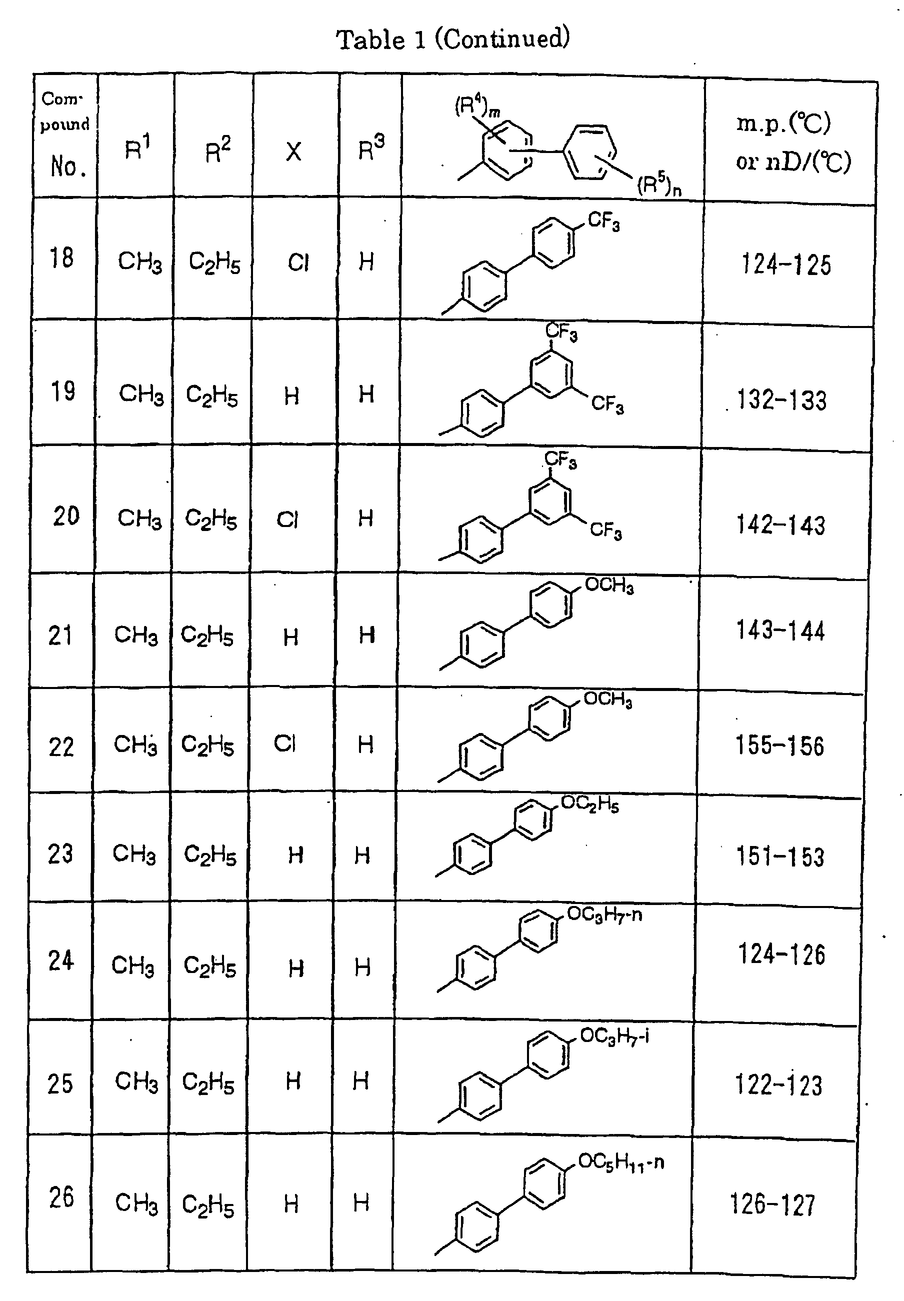

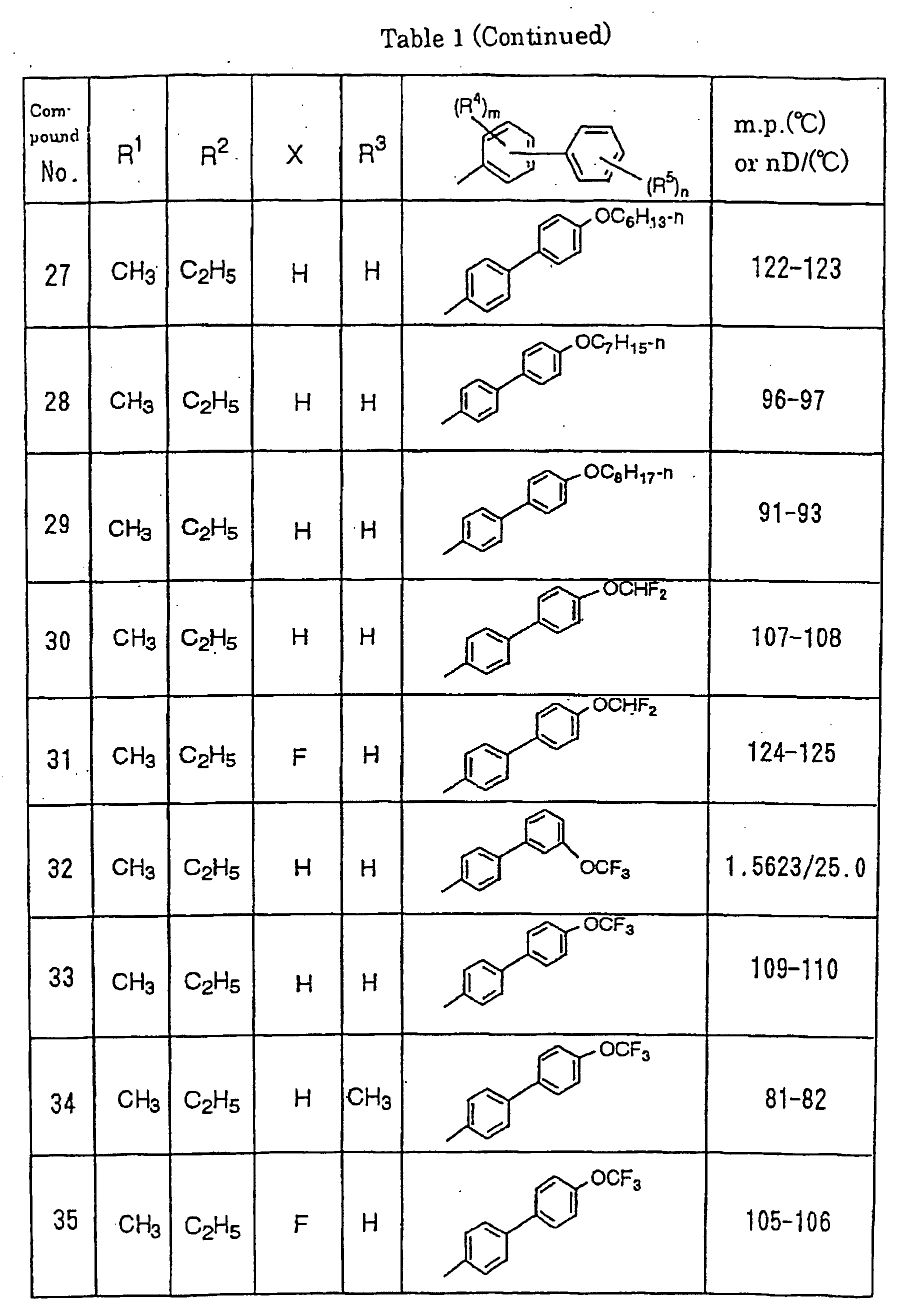

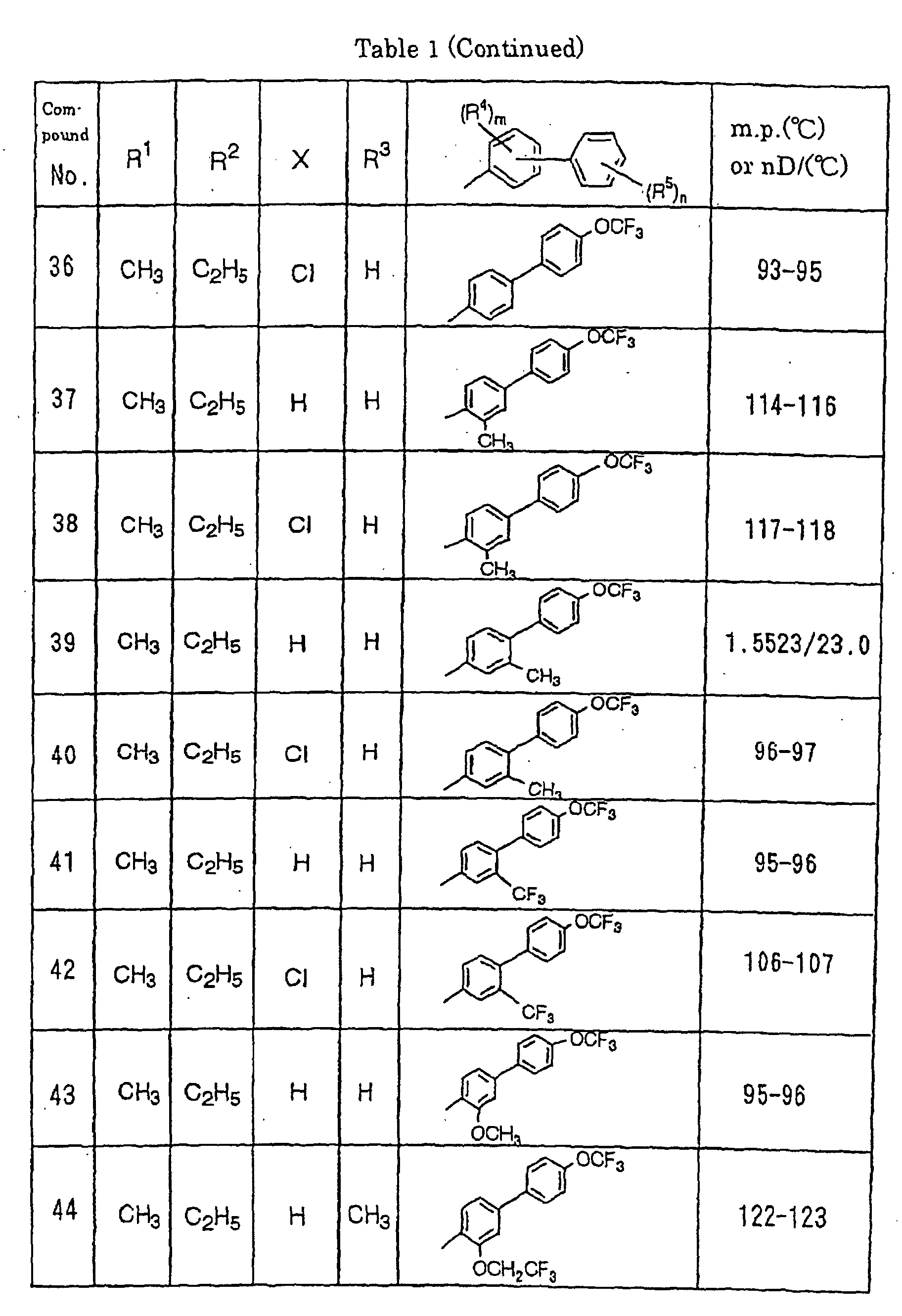

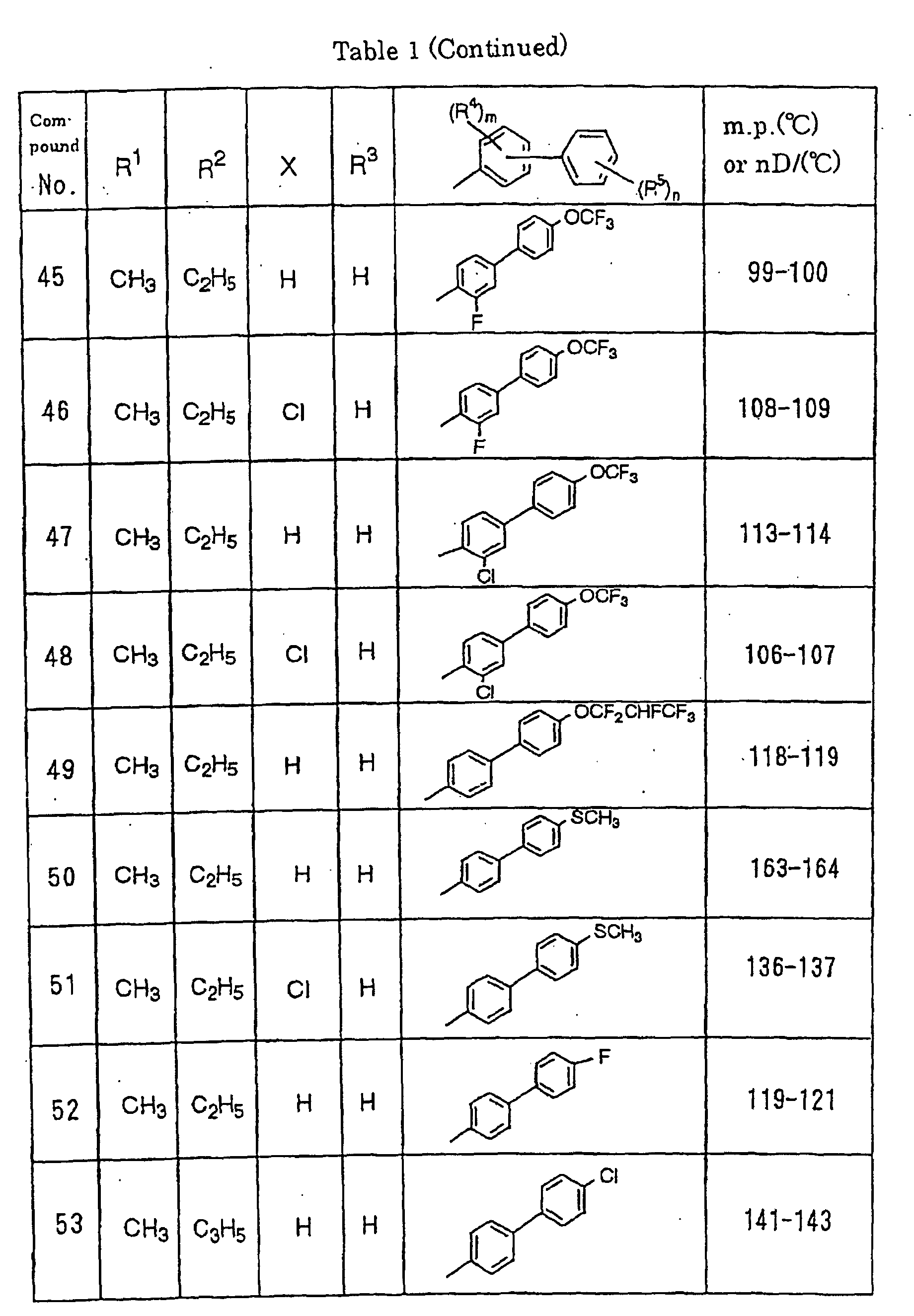

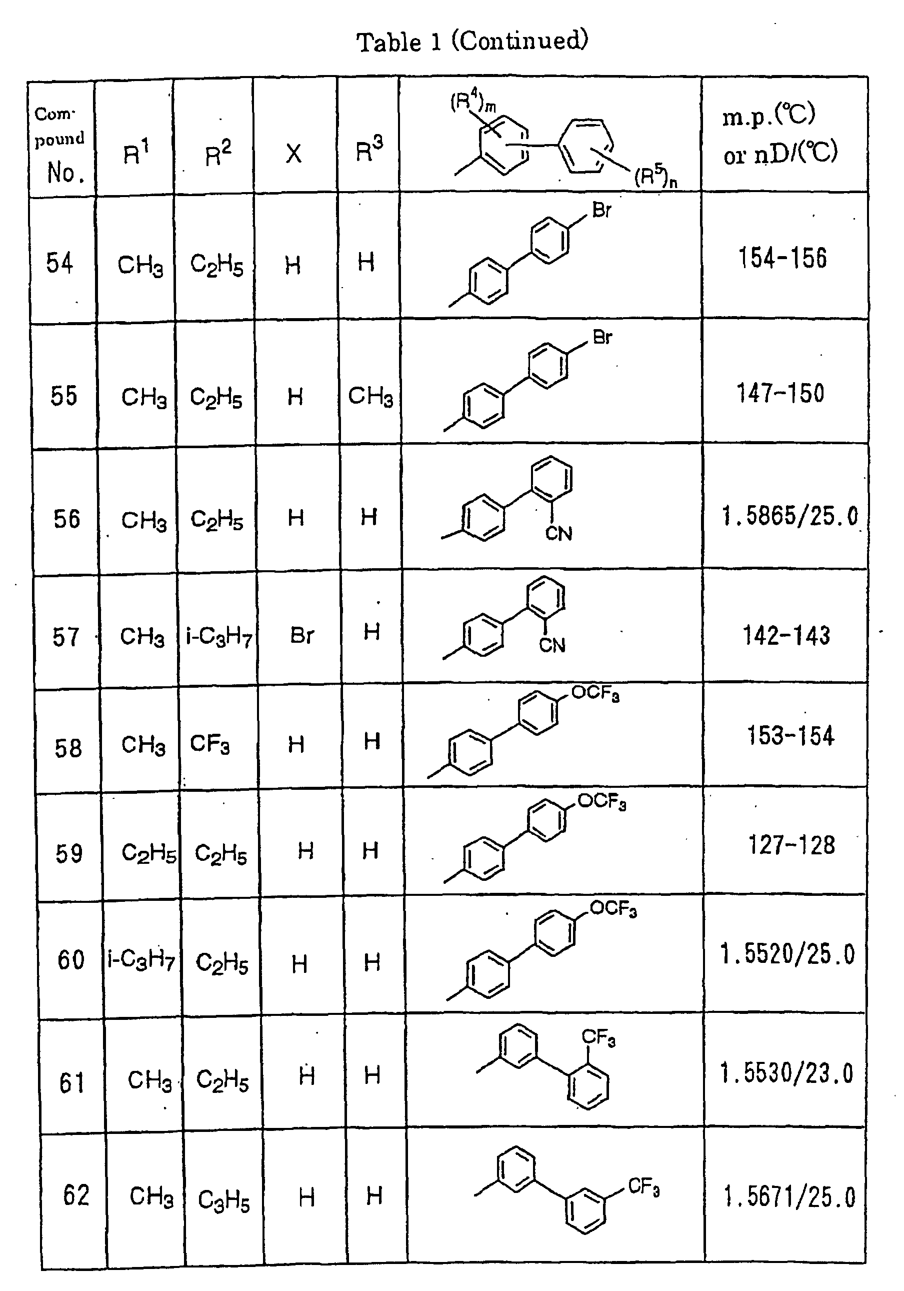

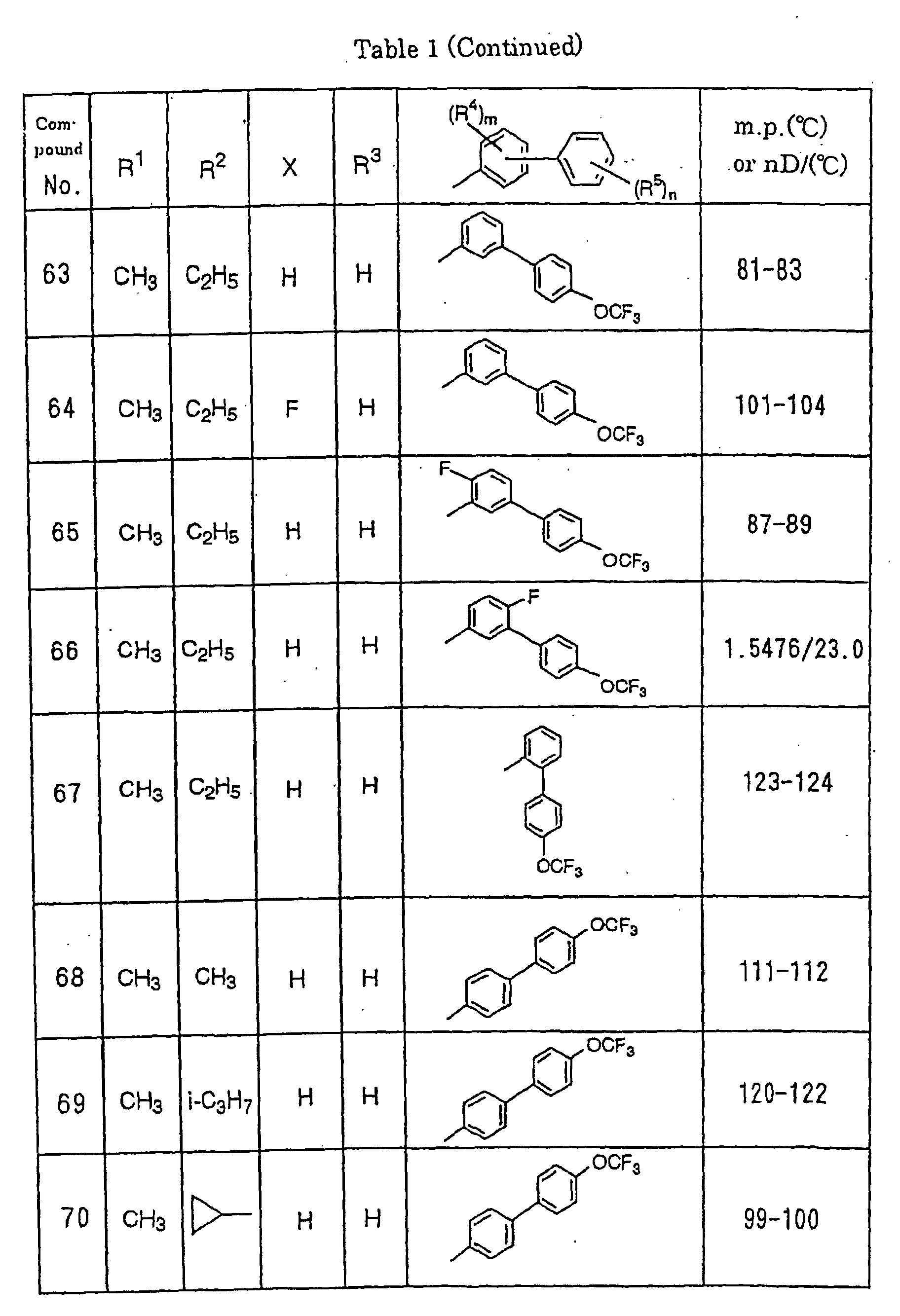

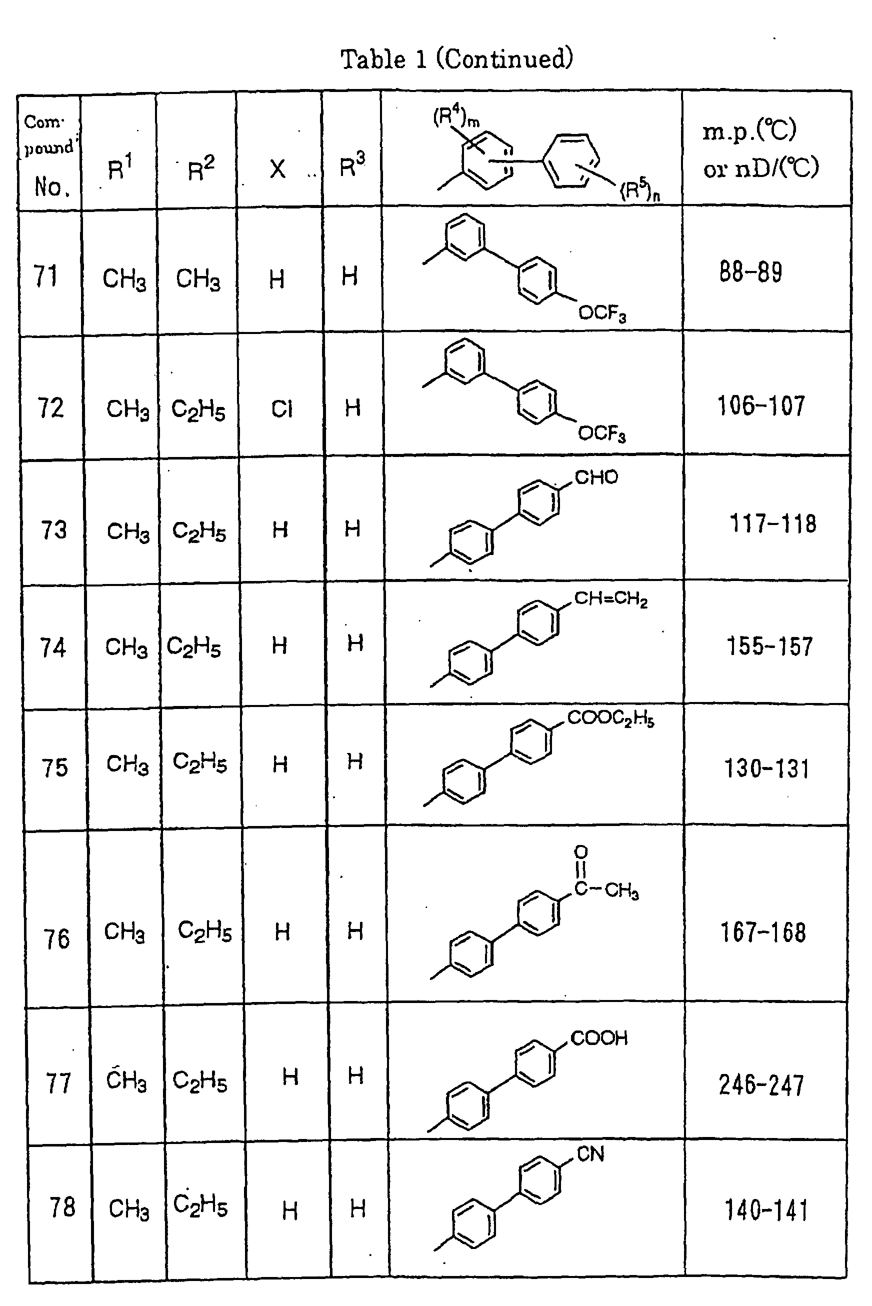

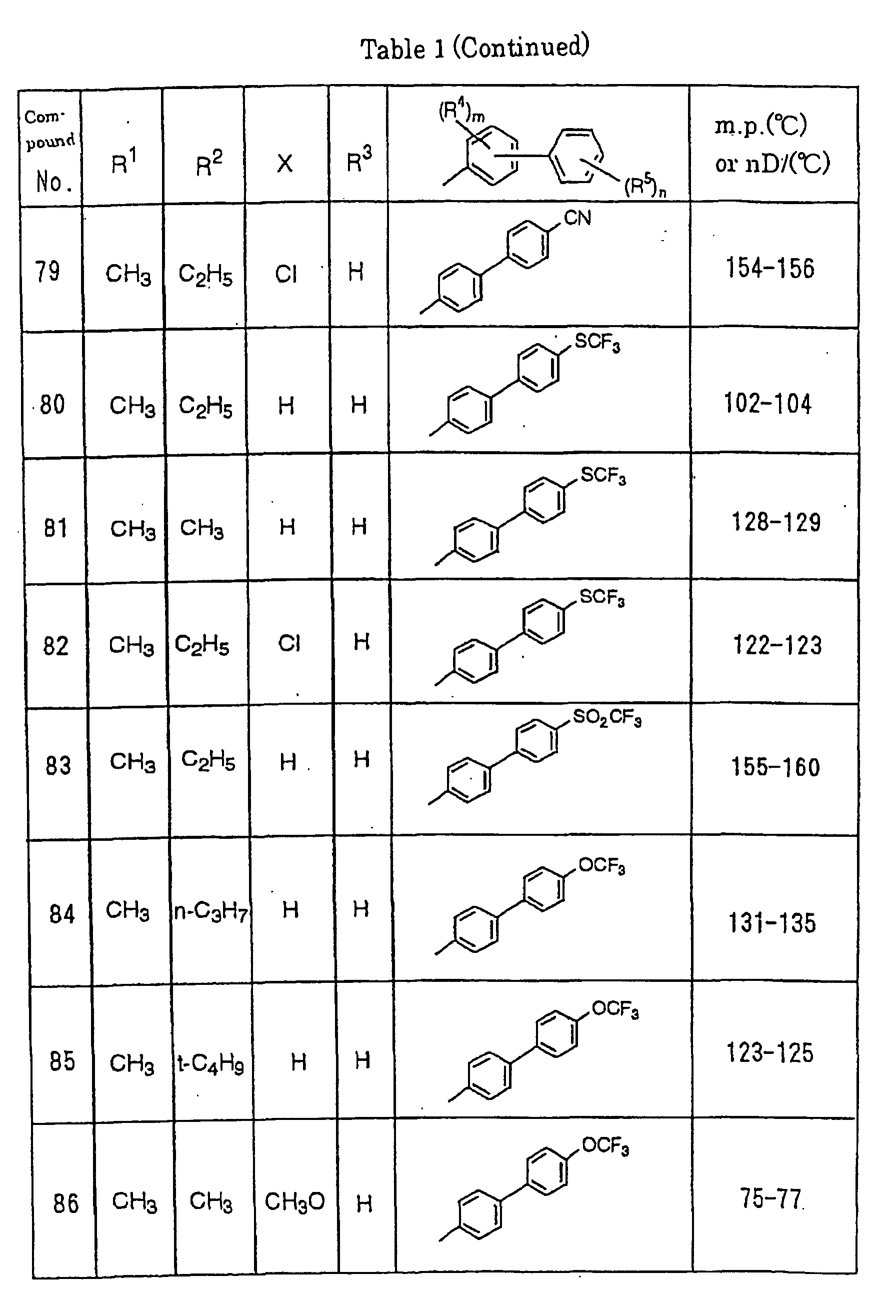

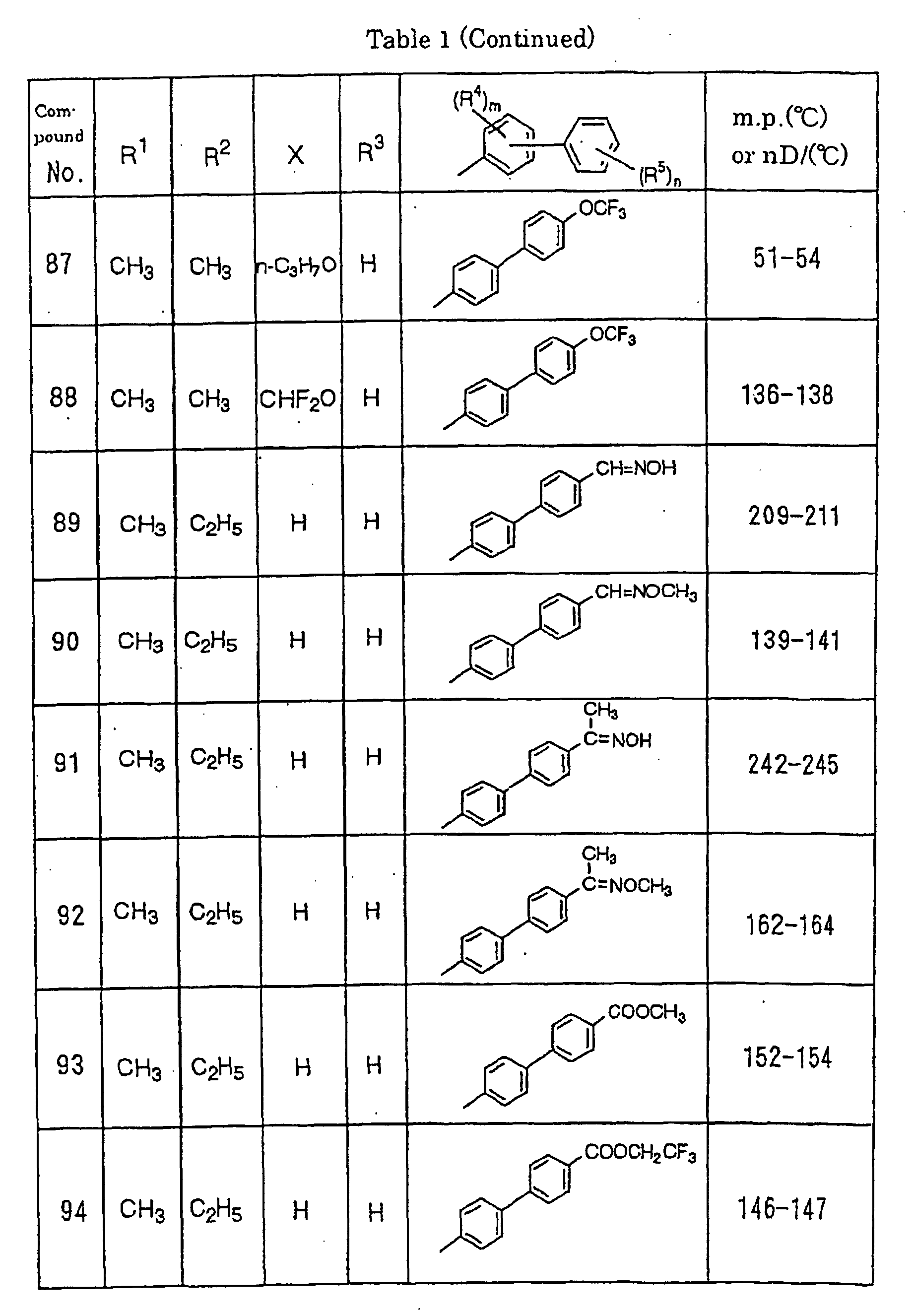

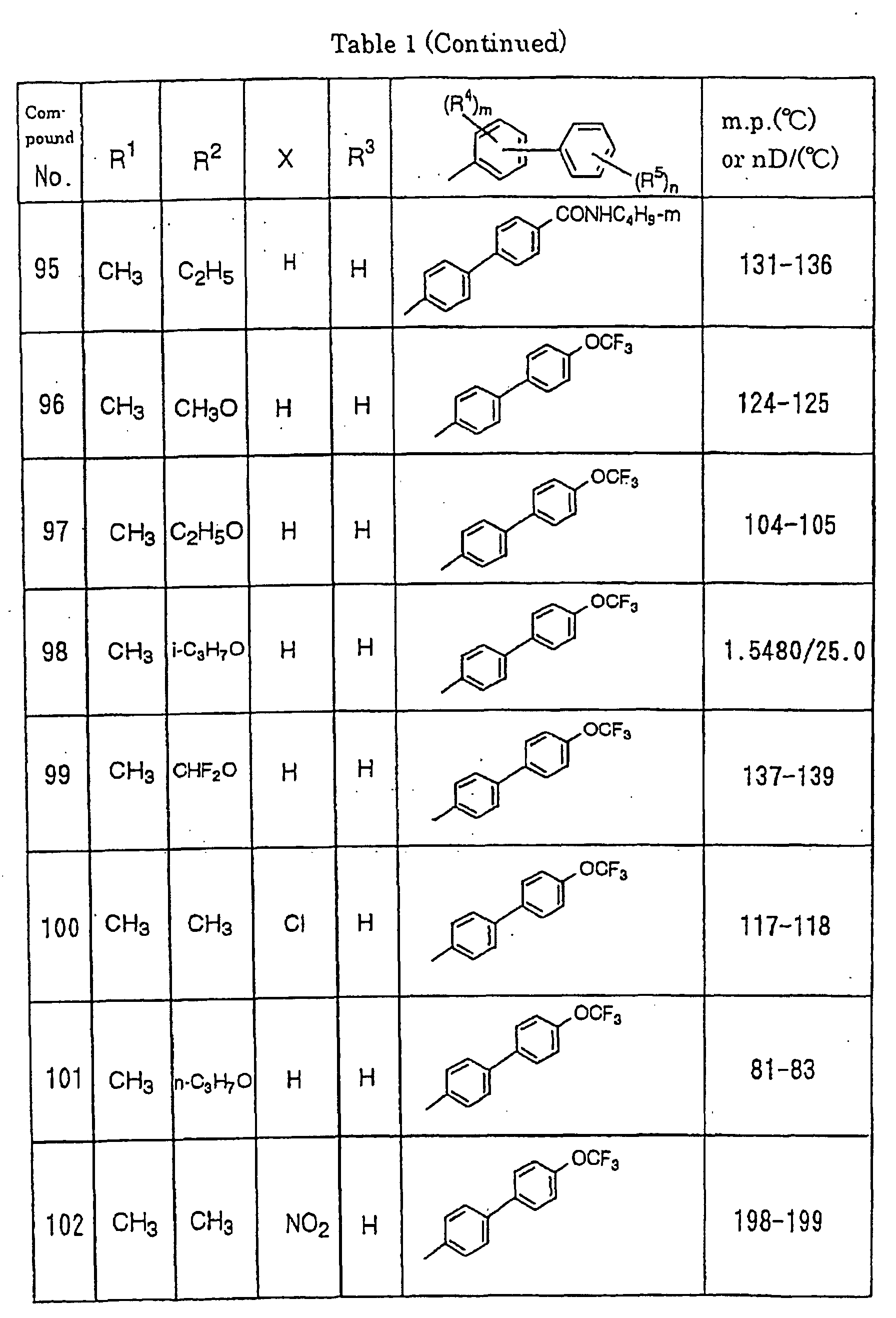

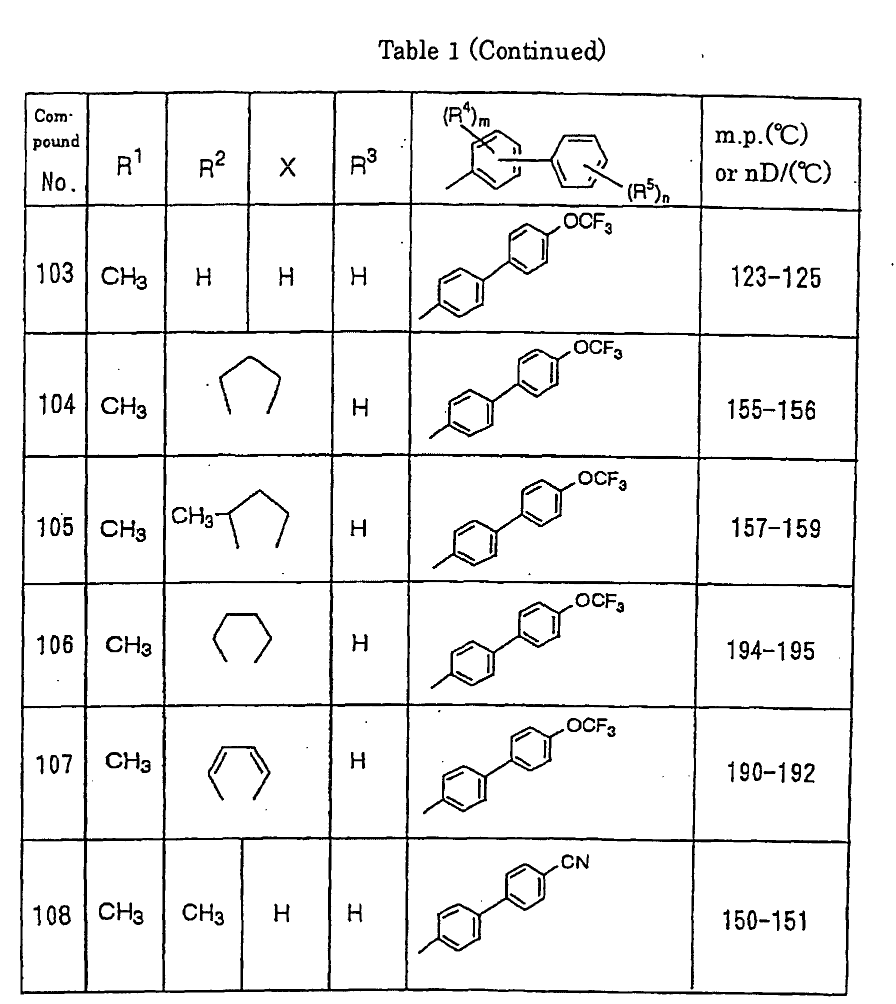

### Example 3

### Synthesis of 4-(4-difluoromethoxyphenyl)benzylamine:

A mixture of 4-cyano-4'-difluoromethoxybiphenyl (15.0 g), Raney-nickel R-100 (15 g) and aqueous 28% ammonia (15.0 ml) was shaken in hydrogen atmosphere at 50°C for 4 hours. After the reaction mixture was cooled to ambient temperature, Raney-nickel was filtered off. The solvent was distilled off under reduced pressure, to obtain 13.8 g of Compound No. 113 described in Table-2. The melting point was 109 to 110°C.

### Example 4

### Synthesis of 4-(4-trifluoromethoxy)-α-methylbenzylamine:

A mixture of 4-(4-trifluoromethoxyphenyl)acetophenone oxime (8.8 g), Raney-nickel R-100 (10 g) and aqueous 28% ammonia (10.0 ml) was shaken in hydrogen atmosphere at 50°C for 3 hours. After the reaction mixture was cooled to ambient temperature, Raney-nickel was filtered off. The solvent was distilled off under reduced pressure, to obtain 7.61 g of Compound No. 117 described in Table-2. The melting point was 70 to 74°C.

### Example 5

### Synthesis of 4-(4-trifluoromethoxyphenyl)benzylamine hydrochloride:

To a solution (20 ml) of 4-(4-trifluoromethoxyphenyl)benzylamine (5 g) in methanol, 12N hydrochloric acid (3.0 ml) was added, followed by agitation at ambient temperature for 2 hours. The solvent was distilled off under reduced pressure. The resulting residue was rinsed with methanol, to obtain 4.8 g of Compound No. 116 described in Table-2. The melting point was 252 to 254°C.

### Example 6

According to the methods described in Examples 3 to 5, the compounds described in Table-2 were synthetically prepared.

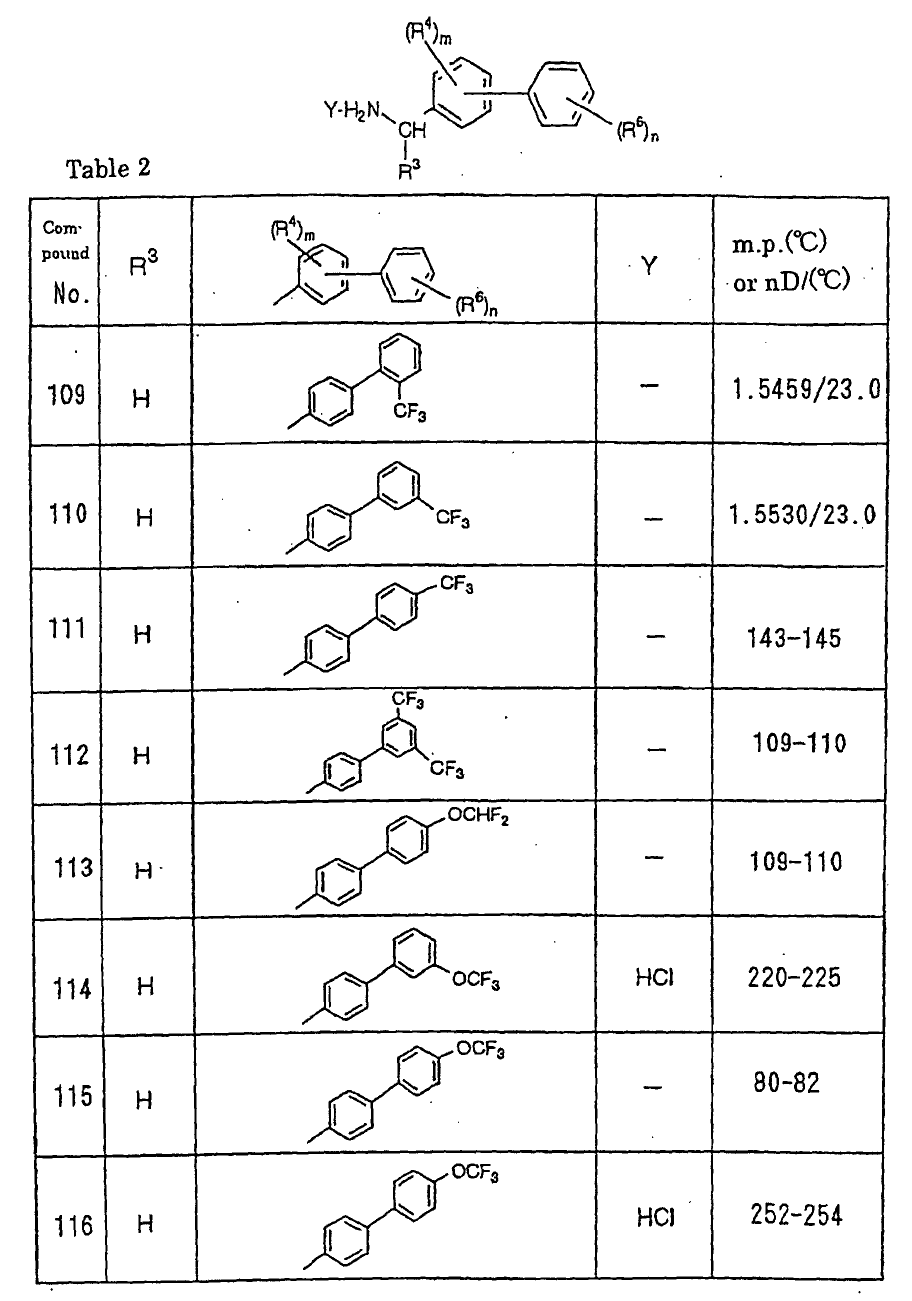

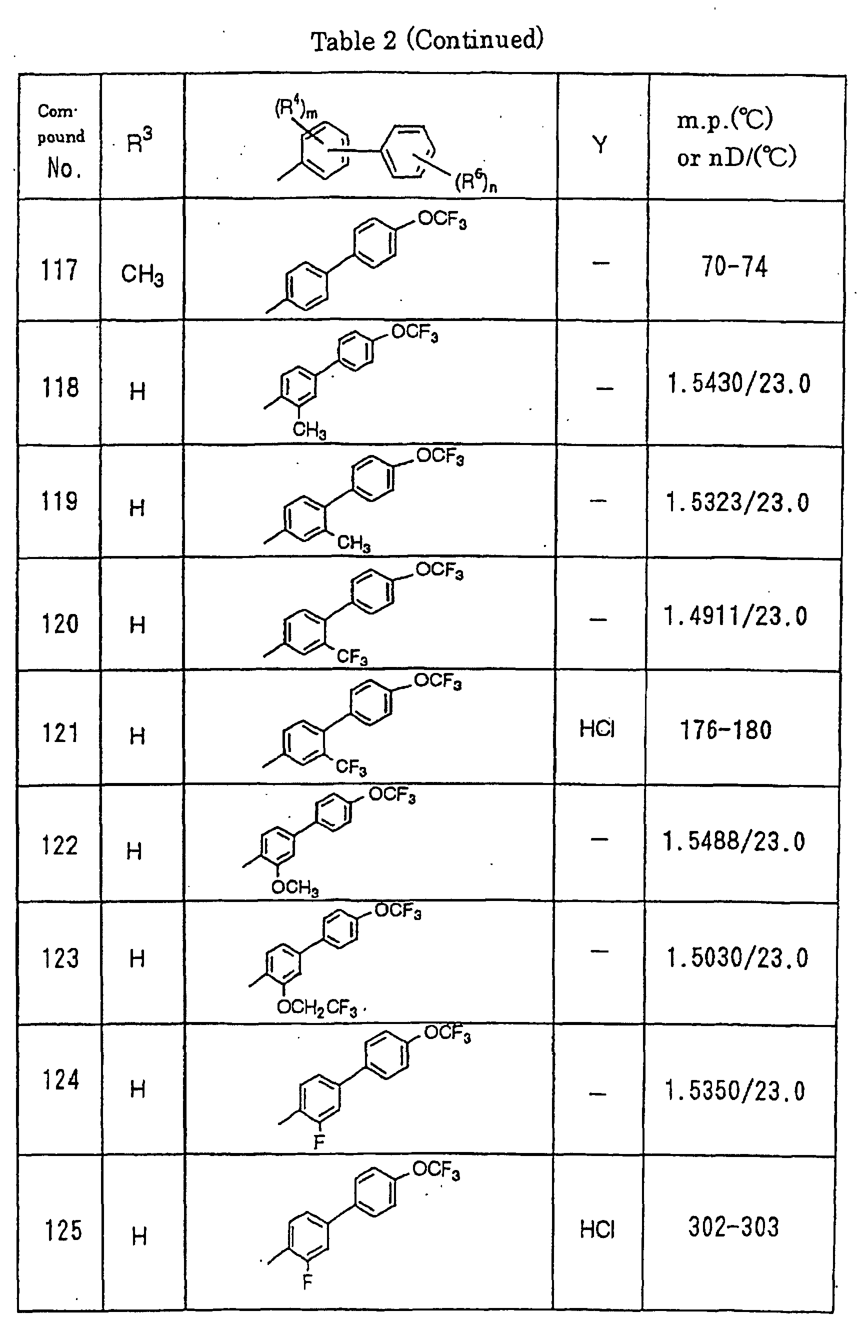

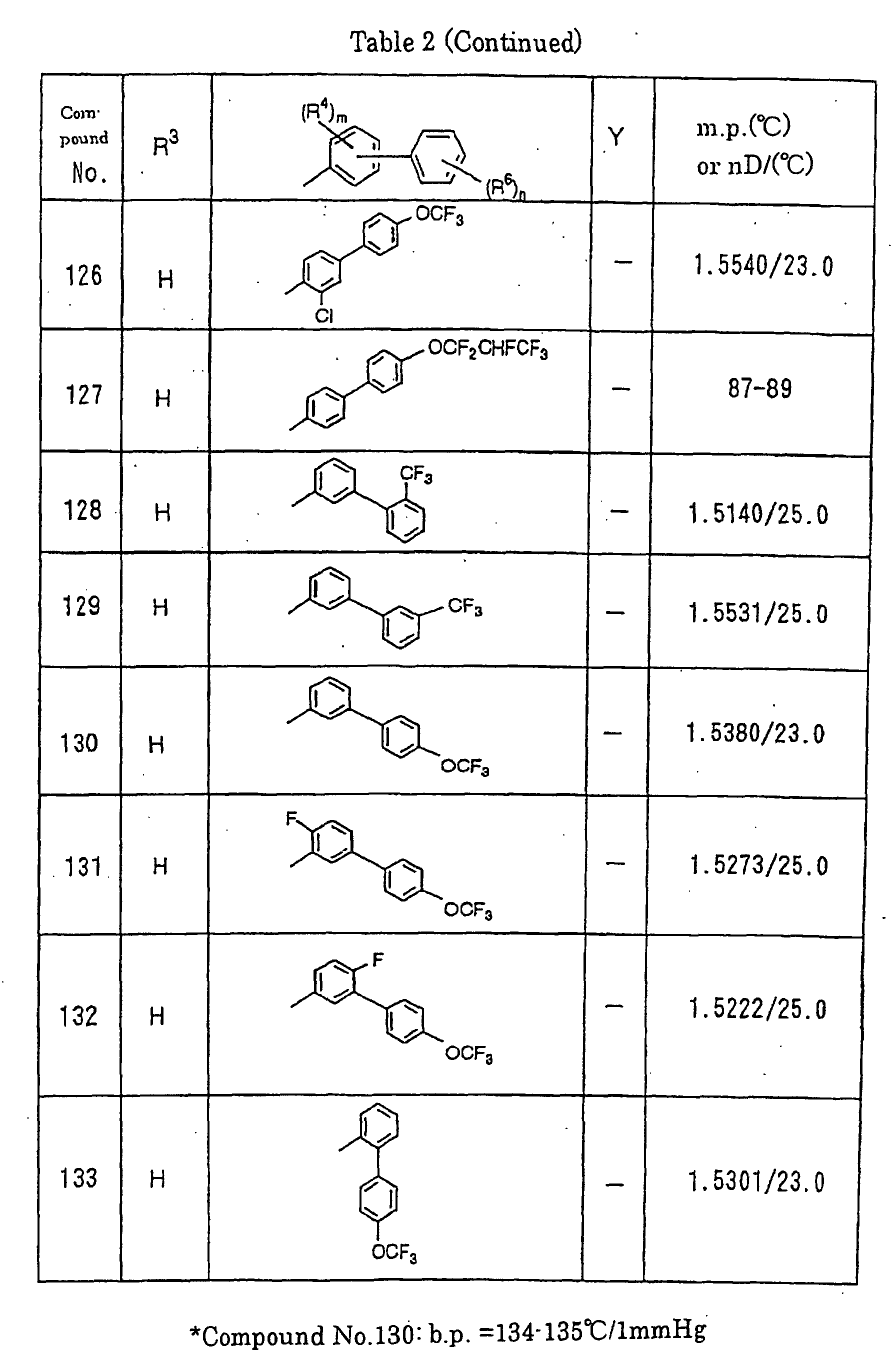

Hereinbelow, Formulation Examples containing the compound of the present invention as the active ingredient are described, which are insecticides and miticides for agriculture and horticulture and floriculture. However, the mode for carrying out the present invention is not limited to those described below.

### Formulation Example 1

### Hydrates:

Twenty parts by weight of the compound of the present invention, 20 parts by weight of Carplex #80 (trade name; white carbon; manufactured by Shionogi & Co., Ltd.), 52 parts by weight of ST kaolin clay (trade name; kaolinite; manufactured by Tuchiya Kaolin Co., Ltd.), 5 parts by weight of Solpol 9047K (trade name; anionic surfactant; manufactured by Toho Chemical, Co., Ltd.) and 3 parts by weight of Lunox P56L (trade name; anionic surfactant; Toho Chemical Co., Ltd.) were blended together, uniformly mixed together and ground, to obtain hydrates containing the active ingredient at a content of 20% by weight.

### Formulation Example 2

### Powders:

Two parts by weight of the compound of the present invention, 93 parts by weight of clay (manufactured by Nippon Talc Co., Ltd.), and 5 parts by weight of Carplex #80 (trade name; white carbon; manufactured by Shionogi Pharmaceutical Co., Ltd.) were uniformly mixed together and ground, to obtain powders containing the active ingredient at a content of 2% by weight.

### Formulation Example 3

### Emulsions:

Twenty parts by weight of the compound of the present invention were dissolved in a mixture solvent of 35 parts by weight of xylene and 30 parts by weight of dimethylformamide, followed by addition of 15 parts by weight of Solpol 3005X (trade name; a mixture of a nonionic surfactant and an anionic surfactant; Toho Chemical Co., Ltd.), to obtain emulsions containing the active ingredient at a content of 20% by weight.

### Formulation Example 4

### Flowable agents:

Thirty parts by weight of the compound of the present invention, 5 parts by weight of Solpol 9047K, 3 parts by weight of Sorbon T-20 (trade name; nonionic surfactant; Toho Pharmaceutical Co., Ltd.), 8 parts by weight of ethylene glycol and 44 parts by weight of water were ground by wet process with Dinomill (manufactured by Sinmaru Enterprise, Co., Ltd.). Ten parts by weight of an aqueous 1% by weight solution of xanthan gum (naturally occurring polymer) were added to the resulting slurry mixture, followed by thorough mixing and grinding, to obtain flowable agents containing the active ingredient at a content of 20% by weight.

Test examples of fungicides and bactericides, insecticides and miticides for agriculture and horticulture and floriculture are shown below, which contain the compound of the present invention as the active ingredient. However, the mode for carrying out the present invention is not limited to those described below.

### Test Example 1

### Fungicidal and bactericidal effect on wheat powdery mildew:

Compound (III) disclosed in JP-A-64-25763, tebufenpyrade and various compounds in accordance with the present invention were individually prepared into preparations in the same manner as in Formulation 3. The resulting preparations were diluted to given concentrations and sprayed on the stems and leave of a wheat plant (species: Norin No.61) at the one-leaf to 2-leaf stage as grown in a pot of a 6-cm diameter, at a ratio of 10-ml volume per one pot. After drying the chemical preparations in air, the wheat plant was exposed to the spore collected from wheat leave afflicted with *Erypsiphe graminis* as the microorganism causing wheat powdery mildew, for inoculation. Then, the plant was left to stand alone in a green house for 7 to 10 days.

Evaluation was carried out follows. The ratio of the diseased area of each leaf was examined to calculate the control ratio according to the following formula. The results are shown in Table 3 (in the table, Compound Nos. correspond to Compound Nos. in Table-1.)
Control ratio (%)
= [(mean ratio of diseased areas in non-treated lot)
- (mean ratio of diseased areas in treated lot)] × 100
/ (mean ratio of diseased areas in non-treated lot)

**Table 3**

| Compound No. | Concentration (ppm) | Control Ratio (%) |
|---|---|---|
| 3 | 500 | 100 |
| 4 | 500 | 100 |
| 5 | 500 | 100 |
| 6 | 500 | 100 |
| 7 | 500 | 100 |
| 13 | 500 | 100 |
| 17 | 500 | 100 |
| 21 | 500 | 100 |
| 25 | 500 | 100 |
| 30 | 500 | 100 |
| 31 | 500 | 100 |
| 34 | 500 | 100 |
| 35 | 500 | 100 |
| 36 | 500 | 100 |
| 37 | 500 | 100 |
| 39 | 500 | 100 |
| 40 | 500 | 100 |
| 41 | 500 | 100 |
| 42 | 500 | 100 |
| 45 | 500 | 100 |
| 46 | 500 | 100 |
| 47 | 500 | 100 |
| 48 | 500 | 100 |
| 49 | 500 | 100 |
| 54 | 500 | 100 |
| 63 | 500 | 100 |
| 64 | 500 | 100 |
| 68 | 500 | 100 |
| 69 | 500 | 100 |
| 70 | 500 | 100 |
| 71 | 500 | 100 |
| 72 | 500 | 100 |
| 78 | 500 | 100 |
| 80 | 500 | 100 |
| 81 | 500 | 100 |
| 82 | 500 | 100 |
| 84 | 500 | 100 |
| 85 | 500 | 100 |
| 86 | 500 | 100 |
| 87 | 500 | 100 |
| 88 | 500 | 100 |
| 90 | 500 | 100 |
| 91 | 500 | 100 |
| 92 | 500 | 100 |
| 98 | 500 | 100 |
| 101 | 500 | 100 |
| (III) | 500 | 0 |
| tebufenpyrade | 500 | 77 |

### Test Example 2

### Fungicidal and bactericidal effect on wheat rust:

Compound (III) disclosed in JP-A-64-25763 and various compounds in accordance with the present invention were individually prepared into preparations in the same manner as in Preparation 3. The resulting preparations were diluted to given concentrations and sprayed on the stems and leave of a wheat seedling (species: Norin No.61) at the one-leaf to 2-leaf stage as grown in a pot of a 6-cm diameter, at a ratio of 10-ml volume per one pot. After drying the chemical preparations in air, a spore suspension prepared by grinding the spore collected from wheat leave afflicted with *Puccinia recondita* as the microorganism causing wheat rust was sprayed on the wheat plant, for inoculation. Then, the plant was left to stand alone in a moist chamber at 22°C for 15 hours. Subsequently, the plant was left to stand in a water pool in a green house for 7 days.

Evaluation was carried out by examining the ratio of the diseased area of each leaf to calculate the control ratio by the same method as in Test Example 1.

The results are shown in Table 4 (in the table, Compound Nos. correspond to Compound Nos. in Table-1.)

**Table 4**

| Compound No. | Concentration (ppm) | Control Ratio (%) |
|---|---|---|
| 3 | 500 | 100 |
| 4 | 500 | 100 |
| 5 | 500 | 100 |
| 6 | 500 | 100 |
| 7 | 500 | 100 |
| 17 | 500 | 100 |
| 21 | 500 | 100 |
| 25 | 500 | 100 |
| 30 | 500 | 100 |
| 34 | 500 | 100 |
| 35 | 500 | 100 |
| 36 | 500 | 100 |
| 39 | 500 | 100 |
| 40 | 500 | 100 |
| 41 | 500 | 100 |
| 45 | 500 | 100 |
| 46 | 500 | 100 |
| 49 | 500 | 100 |
| 54 | 500 | 100 |
| 63 | 500 | 100 |
| 64 | 500 | 100 |
| 68 | 500 | 100 |
| 69 | 500 | 100 |
| 70 | 500 | 100 |
| 71 | 500 | 100 |
| 72 | 500 | 100 |
| 80 | 500 | 100 |
| 86 | 500 | 100 |
| 90 | 500 | 100 |
| (III) | 500 | 0 |

### Test Example 3

### Fungicidal and bactericidal effect on larvae of Spodoptera litura:

Compound (III) disclosed in JP-A-64-25763 and various compounds in accordance with the present invention were individually prepared into insecticide preparations according to the formulation of Formulation Example 1. The resulting insecticides of the present invention (hydrates) were diluted with water, where chopped cabbage leave (a diameter of 6 cm) were then immersed for one minute. After immersion and subsequent drying in air, the cabbage leave were placed in a plastic cup (inner diameter of 7 cm), where five larvae each of 3rd instar *Spodoptera litura* were left (twice in duplicate per one concentration). The plastic cup was kept in a thermostat at 25 °C. Five days after the larvae were left in the plastic cup, the death and agony of these larvae were examined, to calculate the insecticide activity (%) while an agonizing insect was counted as 0.5 insect death. The results are shown in Table 5 (in the table, Compound Nos. correspond to Compound Nos. in Table-1.)

**Table 5**

| Compound No. | Concentration (ppm) | Insecticide Activity (%) |
|---|---|---|
| 3 | 500 | 100 |
| 5 | 500 | 100 |
| 6 | 500 | 100 |
| 7 | 500 | 100 |
| 15 | 500 | 100 |
| 37 | 500 | 100 |
| 39 | 500 | 100 |
| 41 | 500 | 100 |
| 42 | 500 | 100 |
| 47 | 500 | 100 |
| 49 | 500 | 100 |
| 52 | 500 | 100 |
| 55 | 500 | 100 |
| 70 | 500 | 100 |
| 78 | 500 | 100 |
| 86 | 500 | 100 |
| 90 | 500 | 100 |
| 100 | 500 | 100 |
| 107 | 500 | 100 |
| (III) | 500 | 20 |

### Test Example 4

### Miticidal effect on adult Tetranychus urticae Koch

The stem part of a *Phaseolus* seedling with one fresh first leaf remaining thereon was inserted in a test tube (a volume of 50 ml) containing water, where 15 female adult *Tetranychus urticae Koch* per one leaf was then inoculated. One day after the inoculation, the leave deposited with *Tetranychus urticae Koch* were immersed in and treated with an aqueous dilution of each of miticides (emulsions) individually produced from Compound (III) disclosed in JP-A-64-25763, tebufenpyrade and various compounds of the present invention according to the formulation for Formulation Example 3 (for about 5 seconds) (single one concentration, duplicate). The resulting test tubes were kept in a thermostat at 25°C. Five days after the treatment, the number of the female adult *Tetranychus urticae Koch* was counted on the *Phaseolus* leave, to calculate the adult death ratio (%) based on the above results. The results are shown in Table-6 (Compound Nos. in the table below correspond to Compound Nos. in Table-1).

**Table 6**

| Compound No. | Concentration (ppm) | Adult death ratio (%) |
|---|---|---|
| 2 | 500 | 100 |
| 4 | 500 | 100 |
| 5 | 500 | 100 |
| 6 | 500 | 100 |
| 7 | 500 | 100 |
| 11 | 500 | 100 |
| 17 | 500 | 100 |
| 18 | 500 | 100 |
| 22 | 500 | 100 |
| 30 | 500 | 100 |
| 31 | 500 | 100 |
| 32 | 500 | 100 |
| 33 | 500 | 100 |
| 35 | 500 | 100 |
| 36 | 500 | 100 |
| 37 | 500 | 100 |
| 38 | 500 | 100 |
| | 500 | 100 |
| | 12.5 | 100 |
| 39 | 3.1 | 100 |
| | 0.8 | 100 |
| 40 | 500 | 100 |
| 41 | 500 | 100 |
| 42 | 500 | 100 |
| 43 | 500 | 100 |
| 46 | 500 | 100 |
| 47 | 500 | 100 |
| 48 | 500 | 100 |
| 49 | 500 | 100 |
| 50 | 500 | 100 |
| 52 | 500 | 100 |
| 53 | 500 | 100 |
| 55 | 500 | 100 |
| 63 | 500 | 100 |
| 68 | 500 | 100 |
| 69 | 500 | 100 |
| 70 | 500 | 100 |
| 71 | 500 | 100 |
| 78 | 500 | 100 |
| 79 | 500 | 100 |
| 80 | 500 | 100 |
| 81 | 500 | 100 |
| 82 | 500 | 100 |
| 83 | 500 | 100 |
| 84 | 500 | 100 |
| 85 | 500 | 100 |
| 86 | 500 | 100 |
| 87 | 500 | 100 |
| 88 | 500 | 100 |
| 96 | 500 | 100 |
| 98 | 500 | 100 |
| 100 | 500 | 100 |
| 104 | 500 | 100 |
| 105 | 500 | 100 |
| 106 | 500 | 100 |
| 107 | 500 | 100 |
| 108 | 500 | 100 |
| (III) | 500 | 15 |
| | 12.5 | 100 |
| tebufenpyrade | 3.1 | 80 |
| | 0.8 | 0 |

While the invention has been described in detail with reference to specific embodiments thereof, it will be apparent to one skilled in the art that various changes and modifications can be made therein without departing from the spirit and scope thereof.

The present application is based on a Japanese patent application filed on April 6, 2001 (Japanese Patent Application 2001-108116), the entire contents of which are incorporated hereinto by reference.

### Industrial Applicability

The pyrazolecarboxamides of the present invention have such great effects on the control of bacterial strains, hazardous insects and mites that the pyrazolecarboxamides function as great insecticides and miticides for agriculture, forestry and disease control. Additionally, the pyrazolecarboxamides of the present invention are promising as a control agent of various hazardous biological organisms for livestock industry, fishery, or storage of various products and public hygiene.

## Claims

1. Pyrazolecarboxamide represented by the following formula (I): 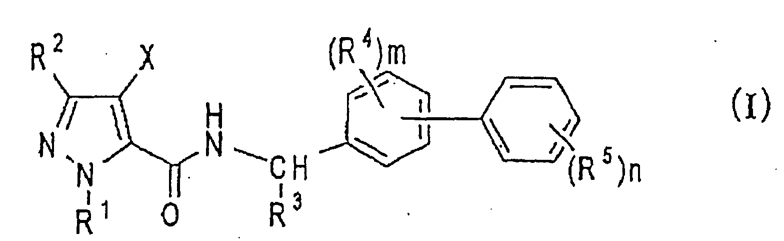 wherein R¹ represents C₁-C₅ alkyl,
R² represents C₁-C₅ alkyl, C₁-C₅ haloalkyl, C₁-C₅ alkoxy or C₁-C₅ haloalkoxy,
R³ represents hydrogen or C₁-C₃ alkyl,
R⁴ represents C₁-C₅ alkyl, C₁-C₅ haloalkyl, C₁-C₅ alkoxy, C₁-C₅ haloalkoxy or halogen,
m is an integer of 0 to 4;
R⁵ represents alkyl, haloalkyl, alkoxy, haloalkoxy, alkylthio, haloalkylthio, alkylsulfinyl, haloalkylsulfinyl, alkylsulfonyl, haloalkylsulfonyl, halogen, cyano, formyl, vinyl, alkoxycarbonyl, haloalkoxycarbonyl, acetyl, hydroxycarbonyl, alkylcarbamoyl or a group represented by 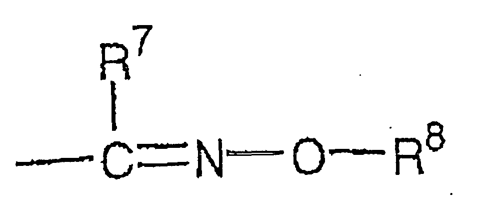 wherein R⁷ and R⁸ each represents hydrogen or C₁-C₅ alkyl,
n represents an integer of 1 to 5,
X represents hydrogen, C₁-C₃ alkyl, C₁-C₃ alkoxy, halogen, C₁-C₃ haloalkoxy or nitro, or
R² and X may be taken together to form 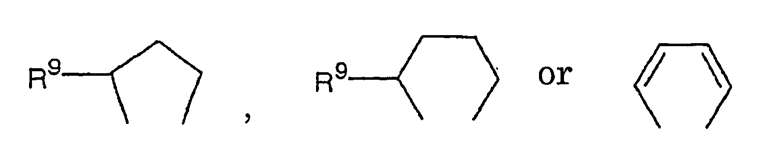 wherein R⁹ represents hydrogen or C₁-C₃ alkyl.

2. The pyrazolecarboxamide according to claim 1, wherein R¹ in formula (I) is methyl.

3. The pyrazolecarboxamide according to claim 1 or 2, wherein R² in formula (I) is ethyl.

4. The pyrazolecarboxamide according to any one of claims 1 to 3, wherein R⁵ in formula (I) is C₁-C₅ haloalkyl or C₁-C₅ haloalkoxy.

5. The pyrazolecarboxamide according to any one of claims 1 to 4, wherein R³ in formula (I) is hydrogen.

6. The pyrazolecarboxamide according to any one of claims 1 to 5, wherein X in formula (I) is hydrogen.

7. The pyrazolecarboxamide according to any one of claims 1 to 6, wherein, in formula (I), m is an integer of 0 to 2 and n is 1 or 2.

8. Phenylbenzylamine represented by the following formula (II): 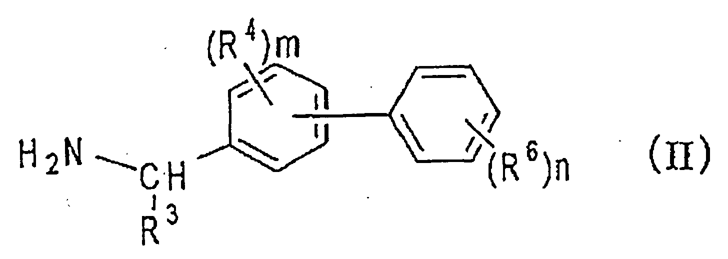 wherein R³, R⁴, m and n are have the same meanings as defined in formula (I); and R⁶ represents haloalkyl or haloalkoxy, or
an acid addition salt thereof.

9. A pest control agent comprising the pyrazolecarboxamide according to any one of claims 1 to 7 as an active ingredient.

10. A fungicidal and bactericidal agent comprising the pyrazolecarboxamide according to any one of claims 1 to 7 as an active ingredient.

11. An insecticidal and miticidal agent comprising the pyrazolecarboxamide according to any one of claims 1 to 7 as an active ingredient thereof.
